# EUROPEAN PATENT SPECIFICATION

(11) **EP 4 645 285 B1**
(45) Date of publication and mention of the grant of the patent: **11.11.2026**
(21) Application number: 25173551.0
(22) Date of filing: 30.04.2025
(51) Int. Cl.: G09B 23/28

(54) **ENDOSCOPIC SURGERY TRAINING DEVICE**
TRAININGSVORRICHTUNG FÜR ENDOSKOPISCHE CHIRURGIE
DISPOSITIF D'ENTRAÎNEMENT CHIRURGICAL ENDOSCOPIQUE

(30) Priority: 30.04.2024 KR 20240057621; 14.05.2024 KR 20240063057; 21.04.2025 KR 20250051715
(43) Date of publication of application: 05.11.2025
(73) Proprietor: Endorobotics Co., Ltd., Seoul 02841 (KR)
(72) Inventor: MIN, Byung Doo, 02577 Seoul (KR); SEO, Yong Tae, 02577 Seoul (KR); SEO, Ye Chan, 02577 Seoul (KR); BILEGT, Erkhes, 02577 Soul (KR); HWANG, Yong Hyun, 02577 Seou (KR); KIM, Jeong Han, 02577 Seoul (KR); CHOI, Soo Yong, 02577 Soul (KR); KIM, Kyung Nam, 02577 Seoul (KR); KIM, Byung Gon, 02577 Seoul (KR)
(74) Representative: Herrmann, Daniel

(56) References cited:
- KR-B1- 102 537 190
- US-A1- 2023 210 350

## Description

### TECHNICAL FIELD

The present invention relates to an endoscopic surgery training apparatus.

### BACKGROUND ART

An endoscopic apparatus is an apparatus that is designed to insert a machine into the body to check for lesions in an organ.

Since endoscopic surgery or procedures using such an endoscopic apparatus are performed in a narrow space inside the human body, a lot of training and experience are required to increase skill.

However, conventional surgical training apparatuses for training endoscopic surgery have problems in that they only provide a model that simulates parts of the body into which the endoscopic apparatus is inserted, and can only train the act of observing and examining lesions inside the body, and cannot provide training for surgery.

KR102537190B1 describes a surgical training device, and more specifically, a surgical training device capable of simulating surgical procedures performed inside the human body using an endoscopic device.

US 2023/210350 A1 describes a medical device operation training apparatus, used in performing training for operation of a medical device, includes: an affected-area simulated organ holding unit configured to hold a sheet-like affected-area simulated organ able to be incised or excised at least in part; and a path simulated organ holding unit configured to hold a path simulated organ for guiding the medical device to the affected-area simulated organ.

### SUMMARY

### TECHNICAL PROBLEM

The present invention has been devised to solve the above problems, and an object of the present invention is to provide an endoscopic surgery training apparatus that can implement a specific part of a target organ similar to an actual organ.

The problems of the present invention are not limited to the problems mentioned above, and other problems that are not mentioned will be clearly understood by those skilled in the art to which the present invention pertains from the description below.

### TECHNICAL SOLUTION

The present invention is defined by independent claim 1. Preferred embodiments are described in the dependent claims, in the description and in the figures.

According to an aspect of the present invention, provided is an endoscopic surgery training apparatus, including a housing including a training space formed inside for training a surgery, and a support plate forming a bottom surface of the training space; a holder configured to fix a training target member; a driving part including a holder support member to which the holder is coupled, and configured to change a position of the holder coupled to the holder support member within the training space through at least one degree-of-freedom movement so as to change the training target member fixed to the holder to a specific simulation position of an organ for training within the training space; a channel forming part formed in the housing so as to form an entry path of an endoscopic device entering the training space from the outside; and a controller for controlling the driving part.

In this case, the driving part may change the position of the holder coupled to the holder support member within the training space through a four-degree-of-freedom movement including a first linear movement, a first rotation, a second rotation and a second linear movement.

In this case, the driving part may include a first linear driving part configured to linearly move the holder support member along the X-axis direction; a first rotational driving part configured to rotate the holder support member around a first rotation axis; a second rotational driving part configured to rotate the holder support member around a second rotation axis; and a second linear driving part configured to linearly move the holder support member in a direction perpendicular to the second rotation axis, and in the holder support member, a first part to which the holder is coupled may be arranged so as to be parallel to the second rotation axis and to be spaced apart from the second rotation axis by a certain distance.

In this case, when the holder support member is placed in an initial position by the driving part while the holder is coupled to the first part, the center of the holder may be placed in a straight line with the first rotation axis, which is the central axis for a first rotation of the holder support member.

Meanwhile, the holder may include a holder body including a mounting part that is detachably coupled to one side of the driving part and a plurality of leg parts that extend from the mounting part, and a plurality of gripping members that are detachably coupled to the plurality of leg parts so as to be able to grip the training target member.

In this case, the holder may include a plurality of first magnetic members that are provided on each of the plurality of leg parts, and a plurality of second magnetic members that are provided on each of the plurality of gripping members so as to correspond to the plurality of first magnetic members, and each of the plurality of gripping members may be detachably coupled to the holder body through the first magnet member and second magnet member corresponding to each other.

Meanwhile, the driving part may include a first linear driving part including a moving member that reciprocates linearly along the X-axis with respect to the support plate via a first driving motor; a first rotational driving part including a second driving motor that is fixedly coupled to the moving member, a first rotation axis that rotates around the Z-axis that is perpendicular to the X-axis with respect to the moving member through a driving force of the second driving motor, and a first mounting member that is fixedly coupled to the first rotation axis; a second rotational driving part including a third driving motor that is fixedly coupled to the first mounting member, a second rotation axis that rotates around an axis that is parallel to the XY plane and perpendicular to the Z axis with respect to the first mounting member through a driving force of the third driving motor, and a second mounting member that is fixedly coupled to the second rotation axis; and a second linear driving part including a fourth driving motor that is fixedly coupled to the second mounting member, and provides a driving force for reciprocating a linear movement of the holder support member along a direction perpendicular to an axis that is parallel to the XY plane and perpendicular to the Z-axis with respect to the second mounting member.

In this case, the endoscopic surgery training apparatus may further include a third magnet member provided on the holder support member, and a fourth magnet member provided on the holder to correspond to the third magnet member, and the holder may be detachably coupled to the holder support member via the third magnetic member and the fourth magnetic member.

Meanwhile, the first linear driving part may further include a first guide rail fixed to the support plate; a first slider movably coupled to the first guide rail so as to be able to move linearly in the X-axis direction along the first guide rail; a first rack gear fixedly coupled to one side of the moving member so as to be parallel to the longitudinal direction of the first guide rail; and a first pinion gear meshed with the first rack gear while being axially coupled to the first driving motor so as to be able to rotate by the first driving motor, and the moving member may move in a reciprocating linear manner along the X-axis with respect to the support plate through the first slider that is fixed to one side.

Meanwhile, the first rotational driving part may further include a first driving gear that is axially coupled to the second driving motor, and a first driven gear that is axially coupled to the first rotation axis and meshes with the first drive gear, and the first mounting member may rotate around the Z-axis, which is perpendicular to the X-axis, with respect to the moving member through a rotation of the first rotation axis.

Meanwhile, the second rotational driving part may further include a second driving gear that is axially coupled to the third driving motor, and a second driven gear that is axially coupled to the second rotation axis and meshes with the second driving gear, and the second mounting member may rotate around an axis perpendicular to the Z-axis with respect to the first mounting member through a rotation of the second rotation axis.

Meanwhile, the second linear driving part may further include a second guide rail fixed to the second mounting member; a second slider movably coupled to the second guide rail so as to be able to reciprocally move linearly along the second guide rail; a second rack gear fixedly coupled to one side of the holder support member so as to be parallel to the longitudinal direction of the second guide rail; and a second pinion gear meshed with the second rack gear while being axially coupled to the fourth driving motor so as to be able to rotate by the fourth driving motor, and the holder support member may move in a reciprocating linear manner along the second guide rail with respect to the second mounting member through the second slider that is fixed to one side.

Meanwhile, the controller may drive the driving part such that the holder support member moves while the training target member is changed to a specific simulation position of an organ for training by driving the driving part.

Meanwhile, the channel forming part may include a channel case coupled to the housing; a path part formed to be recessed from one surface of the channel case so as to form an entry path for the endoscopic device; and a path maintenance member formed in a hollow shape such that the endoscopic device passes through and detachably coupled to the path part, wherein the path part may include a first path forming part formed to be connected to the training space and have a certain length; and a second path forming part and a third path forming part each extending from the first path forming part so as to branch in different directions from the other end of the first path forming part, and wherein the path maintenance member may be mounted on the path part so as to be positioned at the first path forming part and the second path forming part to form a first entry path, or may be mounted on the path part so as to be positioned at the first path forming part and the third path forming part to form a second entry path.

In this case, the path maintenance member may include a hollow connecting pipe having a predetermined length, a first coupling port provided at one end of the connecting pipe so as to be detachably coupled to one end of the first path forming part, and a second coupling port provided at the other end of the connecting pipe so as to be detachably coupled to one end of the second path forming part or one end of the third path forming part.

Meanwhile, while the holder is changed to a specific simulation position of an organ through the driving part, the training target member that is mounted on the holder may implement an inner wall of the organ, and an endoscopic device that enters the training space through the channel forming part may be positioned such that a tip faces one surface of the training target member.

Meanwhile, the endoscopic surgery training apparatus may further include an auxiliary support part positioned at a bottom of the channel forming part so as to support a portion of the endoscopic device that has entered the training space through the channel forming part.

In this case, the auxiliary support part may include an auxiliary support configured to support a portion of an endoscopic device that has entered the training space, and an auxiliary driving part configured to change a position of the auxiliary support through a fifth driving motor within the training space, and the controller may drive the auxiliary driving part to move the auxiliary support toward the training target member when the training target member changes to a preset specific simulation position.

Meanwhile, the auxiliary driving part may further include a third slider fixed to a coupling member that is coupled to the support plate; a third guide rail that is movably coupled to the third slider so as to be able to move linearly in the X-axis direction and has the auxiliary support fixedly coupled to one side; a third rack gear formed along the X-axis direction on one side of the third guide rail; and a third pinion gear that is axially coupled to the fifth driving motor so as to be able to rotate by the fifth driving motor and meshes with the third rack gear, and the auxiliary support may move toward the training target member through a linear movement of the third guide rail.

Meanwhile, the endoscopic surgery training apparatus may further include an operating part for user operation, wherein the operating part may generate a user input signal to change the training target member to a specific simulation position of an organ.

Meanwhile, the endoscopic surgery training apparatus may further include a connector provided on one side of the housing so as to be electrically connected to the holder support member, and a portion of which is exposed to the outside of the housing and is coupled to a terminal of a cable that is electrically connected to a cautery, and the holder that is coupled to the holder support member may be electrically connected to the connector.

### ADVANTAGEOUS EFFECTS

According to the above configuration, the endoscopic surgery training apparatus according to the present invention can implement a specific part of an organ that is targeted by a training target member similarly to an actual organ. Through this, the user can perform surgical training in an environment similar to an actual environment, thereby increasing the skill level

The effects of the present invention are not limited to the above effects, and should be understood to include all effects that can be inferred from the configuration of the invention described in the detailed description or claims of the present invention.

### BRIEF DESCRIPTION OF DRAWINGS

FIG. 1 is a drawing showing an endoscopic surgery training apparatus according to an embodiment of the present invention.
FIG. 2 is a drawing showing a state in which a door is opened and a channel case is withdrawn in FIG. 1.
FIG. 3 is a drawing schematically showing the interior of FIG. 1.
FIG. 4 is a drawing schematically showing the arrangement relationship of a driving part, an auxiliary driving part and a channel forming part arranged inside the housing in FIG. 1.
FIG. 5 is a block diagram showing the main components controlled by a controller in the endoscopic surgery training apparatus according to an embodiment of the present invention.
FIG. 6 is a drawing showing a holder that can be applied to the endoscopic surgery training apparatus according to an embodiment of the present invention.
FIG. 7 is a drawing showing a state in which a holder and a holder support member are separated in the endoscopic surgery training apparatus according to an embodiment of the present invention.
FIG. 8 is a drawing showing a support plate, a driving part and an auxiliary driving part extracted from the endoscopic surgery training apparatus according to an embodiment of the present invention.
FIG. 9 is a drawing of a driving part and a holder extracted from the endoscopic surgical training apparatus according to an embodiment of the present invention, with some of the components separated.
FIG. 10 is a drawing showing a lower part of a moving member in the endoscopic surgery training apparatus according to an embodiment of the present invention.
FIG. 11 is a drawing showing the coupling relationship of a first rotational driving part in the endoscopic surgery training apparatus according to an embodiment of the present invention.
FIG. 12 is a drawing showing the coupling relationship of a second rotational driving part in the endoscopic surgery training apparatus according to an embodiment of the present invention.
FIG. 13 is a drawing showing FIG. 12 from another direction, showing a state in which the second mounting member is removed.
FIG. 14 is a drawing showing the coupling relationship of a second linear driving part in the endoscopic surgery training apparatus according to an embodiment of the present invention.
FIG. 15 is a drawing showing a state in which a holder support member moves linearly through a second linear driving part in the endoscopic surgery training apparatus according to an embodiment of the present invention.
FIG. 16 is a drawing showing a channel forming part that is applicable to the endoscopic surgery training apparatus according to an embodiment of the present invention, showing a state in which a path maintenance member is mounted to be positioned at the first path forming part and the second path forming part.
FIG. 17 is a drawing showing a channel forming part that is applicable to the endoscopic surgery training apparatus according to an embodiment of the present invention, showing a state in which a path maintenance member is mounted to be positioned at the first path forming part and the third path forming part.
FIG. 18 is a drawing showing an auxiliary driving part that is applicable to the endoscopic surgery training apparatus according to an embodiment of the present invention.
FIG. 19 is a drawing looking at FIG. 18 from another direction.
FIG. 20 is a drawing showing a state in which the endoscopic surgery training apparatus according to an embodiment of the present invention is in use, showing a state in which a holder and a training target member are disposed to be positioned at different specific positions.
FIG. 21 is a drawing showing a state in which the endoscopic surgery training apparatus according to an embodiment of the present invention is in use, showing a state in which a holder and a training target member are disposed to be positioned at different specific positions.
FIG. 22 is a diagram showing a state in which the endoscopic surgery training apparatus according to an embodiment of the present invention is in use, showing a state in which a holder and a training target member are disposed to be positioned at other specific positions.

### DETAILED DESCRIPTION

Hereinafter, with reference to the attached drawings, embodiments of the present invention will be described in detail so that those skilled in the art can easily practice the present invention. The present invention may be implemented in various different forms and is not limited to the embodiments described herein. In order to clearly explain the present invention, parts that are not related to the description in the drawings have been omitted, and the same reference numerals have been assigned to the same or similar components throughout the specification.

The words and terms used in the present specification and claims should not be interpreted as having limited meanings in their usual or dictionary sense, but should be interpreted as having meanings and concepts that conform to the technical idea of the present invention according to the principle that the inventor can define terms and concepts in order to explain his or her invention in the best way.

In addition, the X-axis, Y-axis and Z-axis used in the present specification and claims may be three axes that are orthogonal to each other, and the axis perpendicular to the Z-axis may be any straight line that is arranged perpendicular to the Z-axis on the XY plane.

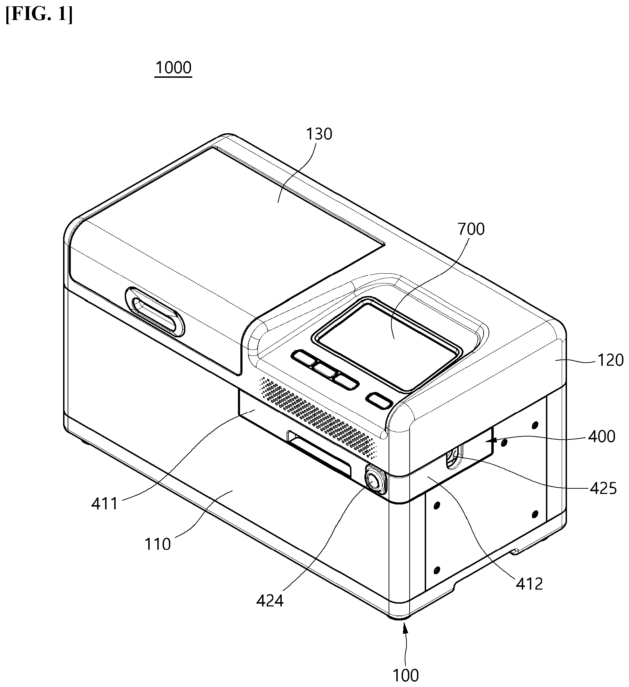

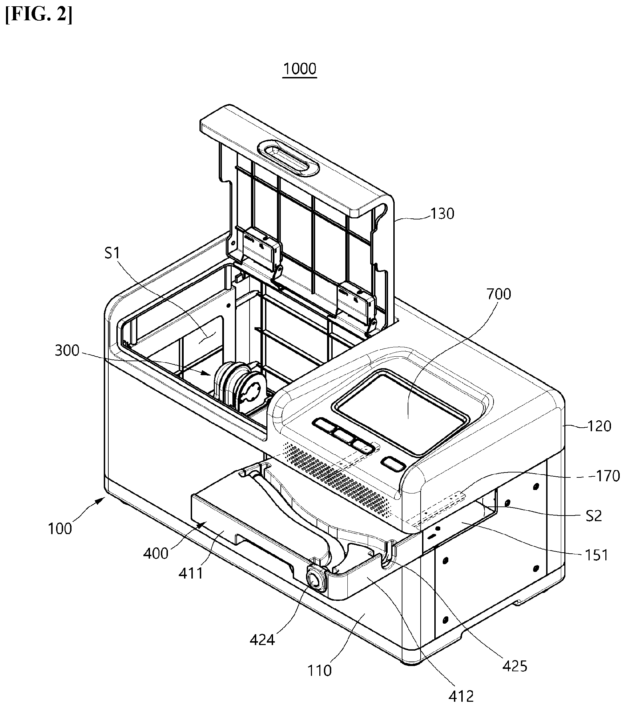

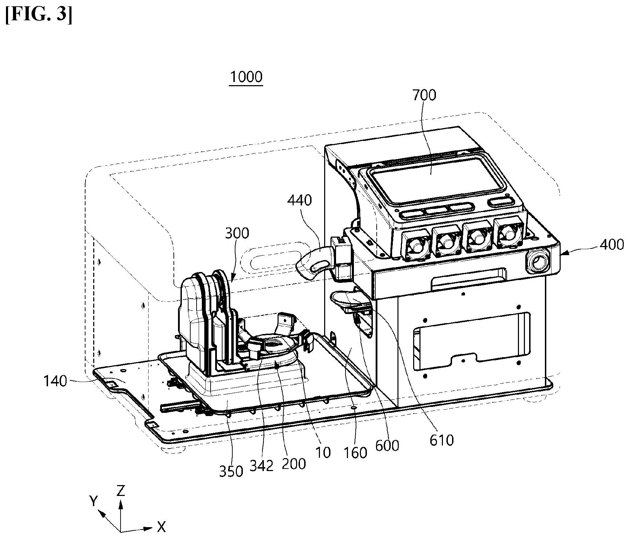

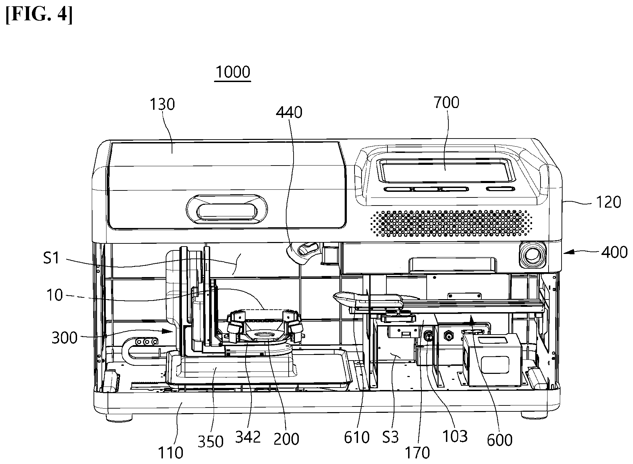

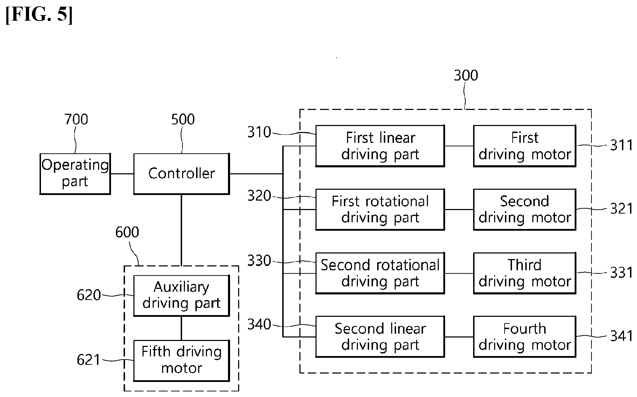

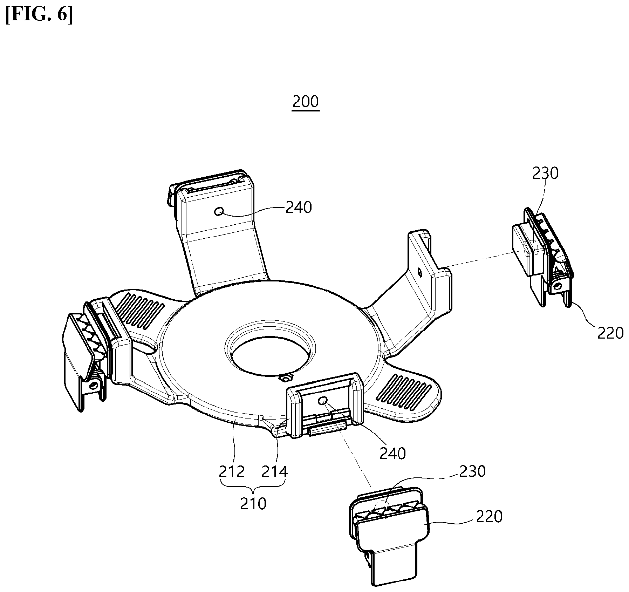

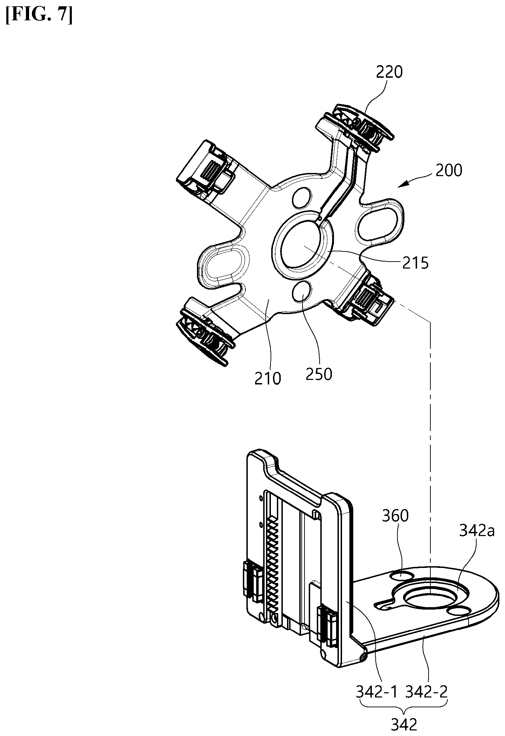

FIG. 1 is a drawing showing an endoscopic surgery training apparatus according to an embodiment of the present invention, FIG. 2 is a drawing showing a state in which a door is opened and a channel case is withdrawn in FIG. 1, FIG. 3 is a drawing schematically showing the interior of FIG. 1, FIG. 4 is a drawing schematically showing the arrangement relationship of a driving part, an auxiliary driving part and a channel forming part arranged inside the housing in FIG. 1, FIG. 5 is a block diagram showing the main components controlled by a controller in the endoscopic surgery training apparatus according to an embodiment of the present invention, FIG. 6 is a drawing showing a holder that can be applied to the endoscopic surgery training apparatus according to an embodiment of the present invention, and FIG. 7 is a drawing showing a state in which a holder and a holder support member are separated in the endoscopic surgery training apparatus according to an embodiment of the present invention.

Referring to FIGS. 1 to 7, the endoscopic surgery training apparatus 1000 according to an embodiment of the present invention may improve the surgical skill of a user, such as a doctor, by implementing an environment similar to an actual surgery.

That is, the endoscopic surgery training apparatus 1000 according to an embodiment of the present invention may implement a specific part of an organ to improve surgical skill by changing a training target member 10 for simulating an organ such as the stomach or the large intestine to a specific position.

For example, the endoscopic surgery training apparatus 1000 according to an embodiment of the present invention may implement a specific part of the stomach or the large intestine by changing the position of the training target member 10, thereby improving the skill of endoscopic surgery for a specific part of the stomach or the large intestine.

The term 'training target member' may be replaced with the term 'training target model'.

The training target member 10 may be formed such that one surface facing the tip of an endoscopic device 20 is formed by a combination of a curved surface and a flat surface, or is formed only by a curved surface, or is formed only by a flat surface.

In the present invention, the training target member 10 may be fixed through a holder 200 described below, and one surface of the training target member 10 facing the tip of the endoscope device 20 in a state where the training target member 10 is changed to a specific position may implement the inner wall of an organ such as the stomach or large intestine.

Herein, the endoscope device 20 may include not only a conventional endoscope device including a camera and a lens module to observe lesions inside the body, but also a surgical tool such as surgical forceps, surgical scissors, a surgical knife, a surgical robot arm that can grasp body tissue or perform a rotational movement and the like that are used in endoscopic surgery or procedures together with the conventional endoscope device.

Accordingly, the user may change the position of the holder 200 to which the training target member 10 is fixed such that the training target member 10 implements a specific part of the target organ, thereby increasing surgical proficiency for a specific part of the organ that requires training.

In addition, the endoscopic surgery training apparatus 1000 according to an embodiment of the present invention is implemented in a portable size such that it can be used in various places without being restricted by location.

To this end, the endoscopic surgery training apparatus 1000 according to an embodiment of the present invention may include a housing 100, a holder 200, a driving part 300, a channel forming part 400 and a controller 500 as shown in FIGS. 1 to 5.

The housing 100 may form an overall outer shape. Such a housing 100 may include a training space (S1) formed inside.

In such a case, the holder 200 and the driving part 300 may be placed in a training space (S1), and the training target member 10 may be fixed to the holder 200.

In addition, the endoscope device 20 may enter the training space (S1) through the channel forming part 400.

Accordingly, the user may perform training on a specific part of an organ requiring training by changing the position of the training target member 10 fixed to the holder 200 through the driving part 300 within the training space (S1).

In this case, the housing 100 may include a door 130 formed in an area corresponding to the training space (S1).

For example, the housing 100 may include a first housing 110 in the shape of a body in which the training space (S1) is formed, a second housing 120 coupled to the first housing 110 so as to cover the training space (S1), and a door 130 openably coupled to the second housing 120 so as to be positioned above the training space (S1).

Accordingly, as shown in FIG. 2, a user may open and close the door 130 to open the training space (S1).

The holder 200 may fix the training target member 10, and may be coupled to one side of the driving part 300.

For example, the holder 200 may be coupled to a holder support member 342 of the driving part 300.

Herein, the holder support member 342 may include a second part 342-1 to which a second slider 344 and a second rack gear 345 described below are fixed, as shown in FIG. 7, and a first part 342-2 to which the holder 200 is detachably coupled.

In this case, as shown in FIG. 6, the holder 200 may include a holder body 210 detachably coupled to the first part 342-2, and a plurality of gripping members 220 coupled to the holder body 210 so as to be able to grip the training target member 10.

In addition, the plurality of gripping members 220 may be arranged on one side of the holder body 210 so as to be spaced apart from each other, and may each grip a portion of the training target member 10.

Herein, the holder body 210 may include a mounting part 212 coupled to the first part 342-2 and a plurality of leg parts 214 extending from the mounting part 212, and the plurality of leg parts 214 may extend from an edge of the mounting part 212 so as to be spaced apart from each other along an edge of the mounting part 212. In addition, the plurality of gripping members 220 may be coupled to the plurality of leg parts 214 so as to correspond one-to-one.

Accordingly, when each of the plurality of gripping members 220 is coupled to the plurality of leg parts 214 while holding the training target member 10, the training target member 10 may be fixed to the holder 200 while maintaining a predetermined shape.

Accordingly, when the position of the first part 342-2 is changed through the driving part 300, the holder 200 coupled to the first part 342-2 may be changed in position together with the first part 342-2, and the training target member 10 fixed to the holder 200 may also be changed in position together with the holder 200.

Through this, the user may change the position of the training target member 10 fixed to the holder 200 within the training space (S1) such that the training target member 10 implements a specific part of the target organ, thereby increasing the surgical skill for the specific part of the organ requiring training.

In this case, each of the plurality of gripping members 220 may be detachably coupled to the plurality of leg parts 214.

For example, each of the plurality of gripping members 220 may be detachably coupled to the plurality of leg parts 214 by using a magnetic force.

To this end, the holder 200 may include a plurality of first magnetic members 230 provided on each of the plurality of leg parts 214 and a plurality of second magnetic members 240 provided on each of the plurality of gripping members 220, as illustrated in FIG. 6.

Herein, each of the first magnet member 230 and the second magnet member 240 may be a permanent magnet.

Accordingly, each of the plurality of gripping members 220 may be detachably coupled to the plurality of leg parts 214 through the corresponding first magnet member 230 and the second magnet member 240.

Through this, the user may use the corresponding first magnet member 230 and the second magnet member 240 to fix the training target member 10 to the holder 200 or easily remove the training target member 10 fixed to the holder 200 from the holder 200.

In this case, the holder 200 may be detachably coupled to one side of the driving part 300 by using a magnetic force similar to the plurality of gripping members 220.

To this end, the endoscopic surgery training apparatus 1000 according to an embodiment of the present invention may include a third magnet member 360 provided on one side of the driving part 300, and a fourth magnet member 250 provided on the holder 200 to correspond to the third magnet member 360.

For example, as shown in FIG. 7, the third magnet member 360 may be provided on the first part 342-2 of the holder support member 342, and the fourth magnet member 250 may be provided on the mounting part 212.

Herein, each of the third magnet member 360 and the fourth magnet member 250 may be a permanent magnet.

Accordingly, the mounting part 212 of the holder 200 may be detachably coupled to the first part 342-2 of the holder support member 342 through the corresponding third magnet member 360 and the fourth magnet member 250.

Through this, the user may use the corresponding third magnet member 360 and the fourth magnet member 250 to fix the holder 200 to the first part 342-2 of the holder support member 342 or easily separate the holder 200 fixed to the first part 342-2 from the first part 342-2.

In the present invention, the holder 200 may be provided to have an appropriate shape depending on the type of the training target member 10 or the organ to be implemented.

Accordingly, the user may perform training by using various training target members 10 by appropriately replacing the holder 200 according to the type or shape of the training target member 10.

In this case, as shown in FIG. 7, the holder 200 may include a protrusion 215 that is formed to protrude from one surface of the mounting part 212, and the holder support member 342 may include a settling groove 342a formed at a position corresponding to the protrusion 215 such that the protrusion 215 can be accommodated.

For example, the settling groove 342a may be formed to be recessed to a certain depth in one surface of the first part 342-2.

Accordingly, when the holder 200 is coupled to the first part 342-2 of the holder support member 342, the protrusion 215 may be inserted into the settling groove 342a.

As a result, when the holder 200 is coupled to the first part 342-2, the holder 200 may be prevented from moving on the first part 342-2 through the mutual coupling of the settling groove 342a and the protrusion 215.

Through this, even if the position of the first part 342-2 is variously changed through the driving part 300, the holder 200 may maintain the initial coupling position with respect to the first part 342-2.

Accordingly, when the position of the first part 342-2 is changed through the driving part 300 while the holder 200 to which the training target member 10 is fixed is coupled to the first part 342-2, the training target member 10 fixed to the holder 200 may be accurately changed to a desired position through a change in the position of the first part 342-2.

The driving part 300 may be disposed to be positioned so as to be positioned within the training space (S1), and the position of the holder support member 342 may be changed in the training space (S1).

Accordingly, the holder 200 coupled to the holder support member 342 may be changed in position through the driving of the driving part 300.

Through this, the driving part 300 may change the training target member 10 fixed to the holder 200 to a specific simulation position of the organ for training within the training space (S1).

That is, the driving part 300 may change the training target member 10 fixed to the holder 200 to a specific position by changing the position of the holder 200 coupled to the holder support member 342, and a specific part of the organ may be implemented by the training target member 10 through a change in position of the training target member 10.

Accordingly, the user may change the position of the training target member 10 fixed to the holder 200 through the driving part 300 while the holder 200 is coupled to the holder support member 342, thereby allowing the training target member 10 to implement a specific part of the intended organ.

In this case, the endoscopic surgery training apparatus 1000 according to an embodiment of the present invention may implement all of various parts of an organ regardless of the shape of the organ.

To this end, the driving part 300 may change the position of the holder 200 coupled to the holder support member 342 in various ways within the training space S1) through a four-degree-of-freedom movement including a first linear movement, a first rotation, a second rotation and a second linear movement.

That is, the endoscopic surgery training apparatus 1000 according to an embodiment of the present invention may enable the holder support member 342 to perform a first linear movement and a second linear movement through the driving part 300, while also enabling a first rotation and a second rotation around different axes.

As a result, the endoscopic surgery training apparatus 1000 according to an embodiment of the present invention may change the position of the holder 200 coupled to the holder support member 342 in various ways through a combination of the first linear movement, the second linear movement, the first rotation and the second rotation of the holder support member 342 through the driving part 300.

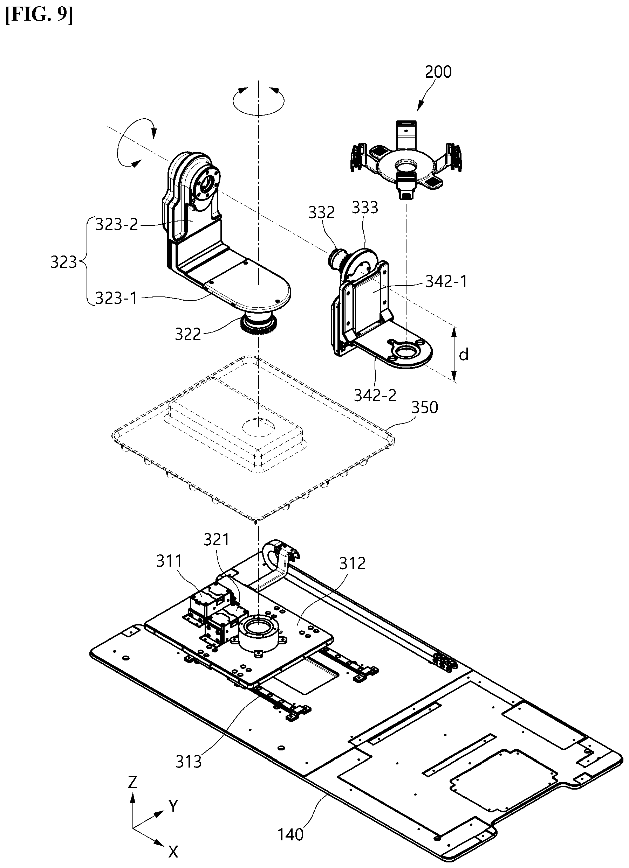

In this case, as shown in FIG. 9, the first part 342-2 of the holder support member 342 to which the holder 200 is coupled may be maintained in a state of being parallel to the second rotation axis 332, which is the central axis for the second rotation of the holder support member 342, and being spaced apart from the second rotation axis 332 by a certain distance (d).

Moreover, the first part 342-2 may move linearly along the X-axis direction together with the first rotation axis 322 and the second rotation axis 332 through the first linear movement, and the separation distance between the first part 342-2 and the second rotation axis 332, which is the central axis of the second rotation, may be adjusted through the second linear movement.

Accordingly, the rotation radius of the first part 342-2 centered on the second rotation axis 332 may be adjusted through the second linear movement of the first part 342-2, and when the first part 342-2 centered on the second rotation axis 332 is rotated at a certain angle, the rotation radius of the first part 342-2 centered on the first rotation axis 322 may be adjusted by the separation distance between the first part 342-2 and the second rotation axis 332.

Accordingly, when the position of the first part 342-2 of the holder support member 342 is changed by the driving part 300 while the holder 200 is coupled to the first part 342-2 of the holder support member 342, the training target member 10 fixed to the holder 200 may implement various parts of the organ regardless of the shape of the organ, and the user may implement a specific part of the desired organ similar to an actual environment by changing the position of the training target member 10 connected to the holder 200.

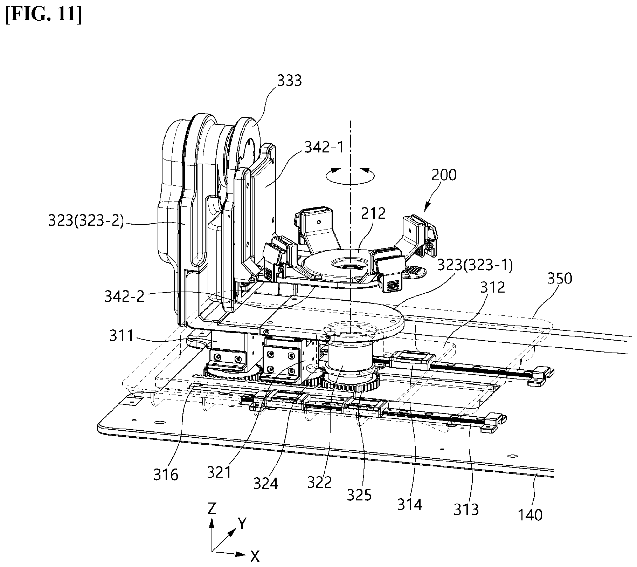

In addition, as shown in FIG. 11, when the holder support member 342 is placed at the initial position by the driving part 300 while the holder 200 is coupled to the holder support member 342, the center of the holder 200 may be arranged to be in a straight line with the first rotation axis 322, which is the central axis for the first rotation of the holder support member 342.

Herein, the center of the holder 200 may be a center point of the mounting part 212 or a center point of the protrusion 215.

Accordingly, when implementing various parts of an organ by changing the position of the training target member 10 fixed to the holder 200, the position of the holder 200 for implementing a specific part of the target organ may be easily calculated, and the first part 342-2 to which the training target member 10 is fixed may be rotated around the two rotation axes 322, 332 to implement the overall shape of the target organ.

Through this, the user may implement a position corresponding to a specific part of the overall shape of the target organ similar to an actual environment by changing the position of the training target member 10 coupled to the holder 200.

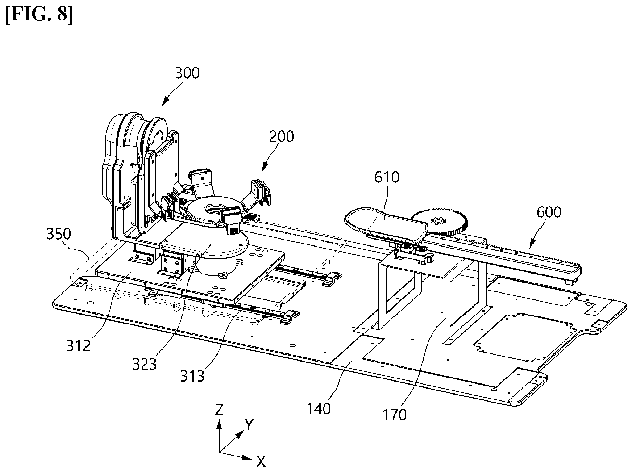

As a non-limiting example, the driving part 300 may include a first linear driving part 310 for the first linear movement of the holder support member 342, a first rotational driving part 320 for the first rotation of the holder support member 342, a second rotational driving part 330 for the second rotation of the holder support member 342, and a second linear driving part 340 for the second linear movement of the holder support member 342, as shown in FIGS. 8 to 14.

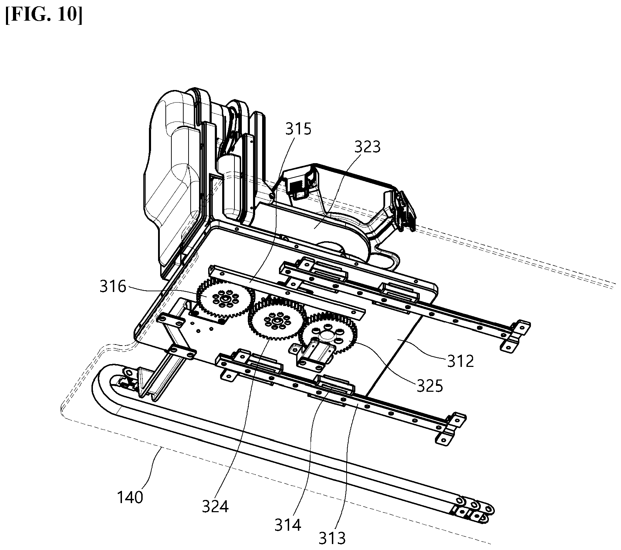

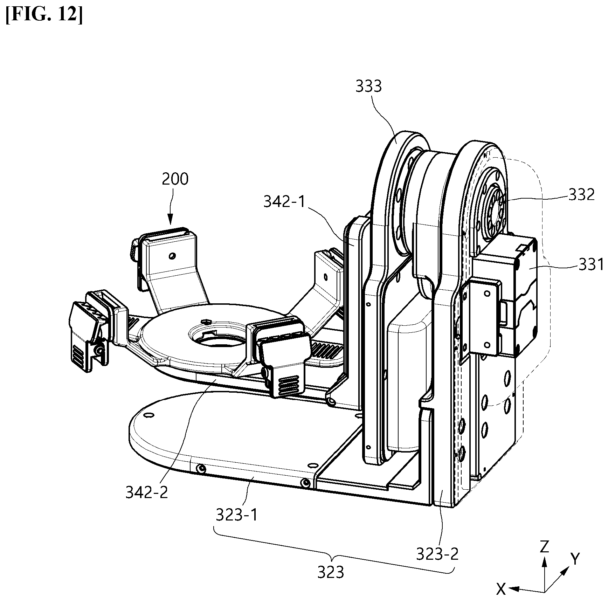

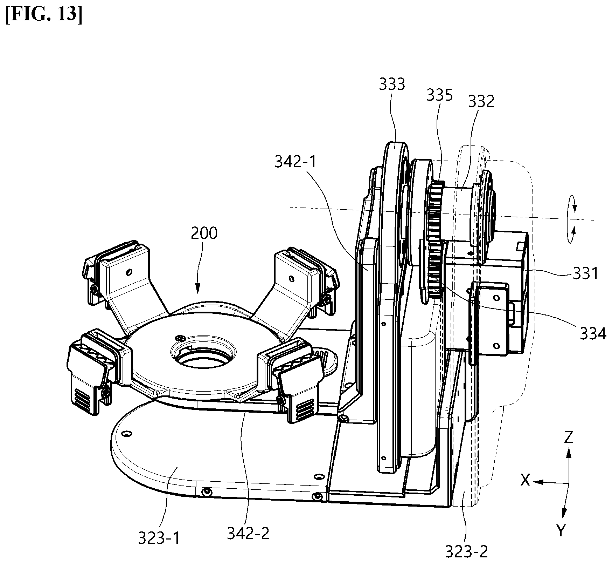

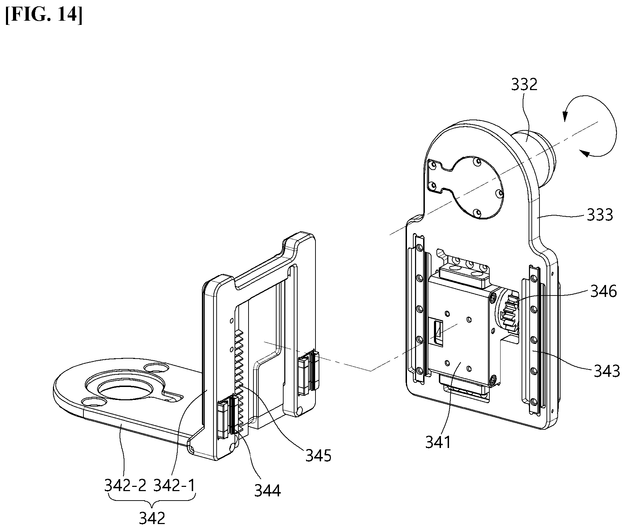

For reference, FIG. 8 is a drawing showing a support plate, a driving part and an auxiliary driving part extracted from the endoscopic surgery training apparatus according to an embodiment of the present invention, FIG. 9 is a drawing of a driving part and a holder extracted from the endoscopic surgical training apparatus according to an embodiment of the present invention, with some of the components separated, FIG. 10 is a drawing showing a lower part of a moving member in the endoscopic surgery training apparatus according to an embodiment of the present invention, FIG. 11 is a drawing showing the coupling relationship of a first rotational driving part in the endoscopic surgery training apparatus according to an embodiment of the present invention, FIG. 12 is a drawing showing the coupling relationship of a second rotational driving part in the endoscopic surgery training apparatus according to an embodiment of the present invention, FIG. 13 is a drawing showing FIG. 12 from another direction, showing a state in which the second mounting member is removed, and FIG. 14 is a drawing showing the coupling relationship of a second linear driving part in the endoscopic surgery training apparatus according to an embodiment of the present invention.

Specifically, the first linear driving part 310 may include a moving member 312 that reciprocates linearly along the X-axis direction with respect to a support plate 140 via a first driving motor 311, as illustrated in FIGS. 10 and 11.

Herein, the support plate 140 may be a plate-shaped member that forms a bottom surface of the training space (S1). In addition, the support plate 140 may be provided as a separate member from the first housing 110, or may be formed integrally with the first housing 110.

In this case, the first linear driving part 310 may further include a first guide rail 313 arranged along the X-axis direction, and a first slider 314 that is movably coupled to the first guide rail 313 so as to be linearly moved along the first guide rail 313.

In addition, the first linear driving part 310 may further include a first rack gear 315 and a first pinion gear 316 that is axially coupled with a rotation axis of the first driving motor 311 so as to be rotated by the first driving motor 311 and meshes with the first rack gear 315.

In this case, the first guide rail 313 may be fixed to one surface of the support plate 140 so as to be arranged parallel to the X-axis direction, the first slider 314 may be coupled to the first guide rail 313 so as to be linearly moved along the first guide rail 313, and the first driving motor 311 may be fixedly coupled to the moving member 312.

In addition, the first rack gear 315 may be fixedly coupled to one side of the moving member 312 so as to be arranged parallel to the X-axis direction, similar to the first guide rail 313.

Accordingly, when the first driving motor 311 rotates through the driving of the controller 500, the first pinion gear 316 may transmit a driving force to the first rack gear 315 through rotation, and the first rack gear 315 may convert a rotational force of the first pinion gear 316 into a driving force for linear movement.

That is, the first rack gear 315 may convert a rotational force of the first pinion gear 316 into a direction parallel to the X-axis.

Herein, the controller 500 may perform a role of controlling the overall driving and may be an MCU or a processor. Moreover, the controller 500 may further include a memory unit (not shown) for storing input information.

Through this, the first slider 314 may reciprocally move linearly along the first guide rail 313 through the driving force provided from the first rack gear 315.

As a result, the moving member 312 to which the first slider 314 is fixed on one side may move linearly along the X-axis direction with respect to the support plate 140. That is, the first linear driving part 310 may implement the first linear movement of the moving member 312.

Referring again to FIGS. 10 and 11, the first rotational driving part 320 may include a second driving motor 321, a first rotational axis 322 that rotates by receiving a rotational force of the second driving motor 321, and a first mounting member 323 that is fixedly coupled to one end side of the first rotational axis 322.

In addition, the first rotational driving part 320 may further include a first driving gear 324 that is axially coupled to a rotational axis of the second driving motor 321), and a first driven gear 325 that is axially coupled to the first rotational axis 322 and meshes with the first driving gear 324.

In this case, the second driving motor 321 may be fixedly coupled to the moving member 312, and the first rotation axis 322 may be arranged parallel to the Z-axis, which is perpendicular to the X-axis, with respect to the moving member 312.

In addition, the first rotation axis 322 may be arranged parallel to the Z-axis such that the center of the holder 200 can be located in a straight line when the holder support member 342 is placed at the initial position while the holder 200 is coupled to the holder support member 342.

Moreover, the first driving gear 324 and the first driven gear 325 may be arranged to be positioned on a lower side of the moving member 312, and the first mounting member 323 fixed to one end side of the first rotational axis 322 may be arranged to be positioned on an upper side of the moving member 312.

Accordingly, when the second driving motor 321 rotates through the driving of the controller 500, the first driving gear 324 may rotate the first driven gear 325, and the first rotational axis 322 may rotate together with the first driven gear 325.

That is, the first rotation axis 322 may rotate around the Z axis that is perpendicular to the X axis with respect to the moving member 312, and the first mounting member 323 fixed to an end side of the first rotation axis 322 may rotate in the same direction as the first rotation axis 322.

As a result, the first mounting member 323 may rotate around the Z-axis that is perpendicular to the X-axis with respect to the moving member 312 through the rotation of the first rotation axis 322. That is, the first mounting member 323 may rotate around the first rotation axis 322 that is arranged parallel to the Z-axis, and the first rotational driving part 320 may implement a first rotation of the first mounting member 323 around the first rotation axis 322.

Herein, the endoscopic surgery training apparatus 1000 according to an embodiment of the present invention may further include a cover member 350 coupled to the moving member 312, and the cover member 350 may be coupled to the moving member 312 so as to cover the first driving motor 311, the second driving motor 321 and the first rotational axis 322 arranged on an upper surface of the moving member 312.

In this case, the first rotational axis 322 may be arranged to penetrate the cover member 350, and the first mounting member 323 may be coupled to the first rotational axis 322 so as to be exposed to the outside from an upper side of the cover member 350. Accordingly, the first mounting member 323 may freely rotate through a rotation of the first rotational axis 322.

Referring again to FIGS. 12 and 13, the second rotational driving part 330 may include the third driving motor 331, a second rotational axis 332 that rotates by receiving a rotational force of the third driving motor 331, and a second mounting member 333 that is fixedly coupled to the second rotational axis 332.

In addition, the second rotational driving part 330 may further include a second driving gear 334 that is axially coupled to a rotational axis of the third driving motor 331, and a second driven gear 335 that is axially coupled to the second rotational axis 332 and meshes with the second driving gear 334.

In this case, the third driving motor 331 may be fixedly coupled to the first mounting member 323, and the second rotation axis 332 may be arranged to be parallel to the XY plane that is perpendicular to the Z-axis, and to be perpendicular to the Z-axis.

In addition, one end part of the second rotation axis 332 may be rotatably coupled to the first mounting member 323, and the other end part of the second rotation axis 332 may be fixed to the second mounting member 333.

Herein, the first mounting member 323 may include a first-1 mounting member 323-1 to which an end of the first rotation axis 322 is fixed, and a first-2 mounting member 323-2 to which an end of the second rotation axis 332 is rotatably coupled.

In this case, the part where the end of the first rotation axis 322 is fixed in the first-1 mounting member 323-1 may be a part that is arranged parallel to the XY plane and perpendicular to the Z-axis, and the part where the end of the second rotation axis 332 is coupled in the first-2 mounting member 323-2 may be a part that is arranged parallel to the Z-axis.

In addition, the second mounting member 333 may be arranged parallel to the first-2 mounting member 323-2 with a gap therebetween, and the third driving motor 331 may be fixed to the first-2 mounting member 323-2.

Accordingly, the second rotation axis 332 may be arranged parallel to the XY plane and perpendicular to the Z-axis, with both ends coupled to the second mounting member 333 and the first-2 mounting member 323-2, respectively.

In addition, the first-1 mounting member 323-1 may be maintained in a state of being spaced apart from the second rotation axis 332 by a certain distance along the Z-axis direction while being parallel to the second rotation axis 332.

Through this, when the third driving motor 331 rotates through the driving of the controller 500, the second driving gear 334 may rotate the second driven gear 335, and the second rotation axis 332 may rotate together with the second driven gear 335.

That is, the second rotation axis 332 may rotate about an axis that is parallel to the XY plane and perpendicular to the Z-axis with respect to the first-2 mounting member 323-2, and the second mounting member 333 fixed to the second rotation axis 332 may rotate in the same direction as the second rotation axis 332.

As a result, the second mounting member 333 may rotate around an axis that is parallel to the XY plane and perpendicular to the Z axis through the rotation of the second rotation axis 332. That is, the second mounting member 333 may rotate around the second rotation axis 332 that is arranged parallel to the XY plane and perpendicular to the Z axis, and the second rotational driving part 330 may implement a second rotation of the second mounting member 333 around the second rotation axis 332.

In other words, the second mounting member 333 may rotate around the second rotation axis 332 that is arranged perpendicular to the first-2 mounting member 323-2.

Referring again to FIG. 14, the second linear driving part 340 may include a fourth driving motor 341 and a holder support member 342 that reciprocally moves linearly with respect to the second mounting member 333 through the fourth driving motor 341.

In addition, the second linear driving part 340 may further include a second guide rail 343 and a second slider 344 that is movably coupled to the second guide rail 343 so as to reciprocally move linearly along the second guide rail 343.

In this case, the second guide rail 343 may be arranged perpendicular to the second rotation axis 332 that is arranged parallel to the XY plane and perpendicular to the Z axis.

Moreover, the second linear driving part 340 may further include a second rack gear 345 arranged parallel to the longitudinal direction of the second guide rail 343, and a second pinion gear 346 that is axially coupled to a rotation axis of the fourth driving motor 341 so as to be rotated by the fourth driving motor 341 and meshes with the second rack gear 345.

In this case, the fourth driving motor 341 may be fixedly coupled to the second mounting member 333, and the second guide rail 343 may be fixed to one surface of the second mounting member 333 so as to be arranged perpendicular to the second rotation axis 332.

In addition, the second slider 344 may be fixed to one surface of the holder support member 342, the second slider 344 may be coupled to the second guide rail 323 so as to move linearly along the second guide rail 343, and the second rack gear 345 may be coupled to one side of the holder support member 342 so as to be arranged parallel to the longitudinal direction of the second guide rail 343 and mesh with the second pinion gear 346.

Herein, as described above, the holder support member 342 may include a second part 342-1 to which the second slider 344 and the second rack gear 345 are fixed, and a first part 342-2 to which the holder 200 is detachably coupled.

In addition, the second part 342-1 may be arranged such that one surface faces the second mounting member 333 as shown in FIG. 12 and FIG. 13, and the first part 342-2 may extend a certain length from the end of the second part 342-1 along a direction parallel to the axial direction of the second rotation axis 332.

That is, the second part 342-1 and the first part 342-2 may be arranged so as to be perpendicular to each other, and the first part 342-2 may be arranged so as to be parallel to the second rotation axis 332. In this case, the third magnet member 360 may be provided on the first part 342-2.

Accordingly, when the holder support member 342 is placed in the initial position through the driving part 300 as shown in FIG. 8, the first part 342-2 may be positioned above the moving member 312 and above the first-1 mounting member 323-1, and may be arranged parallel to both of the moving member 312 and the first-1 mounting member 323-1.

Likewise, when the holder support member 342 is placed in the initial position through the driving part 300 as shown in FIG. 8, the mounting part 212 of the holder 200 coupled to the first part 342-2 may also be positioned above the moving member 312 and above the first-1 mounting member 323-1, and may be arranged parallel to both of the moving member 312 and the first-1 mounting member 323-1.

Accordingly, the first part 342-2 may be arranged parallel to the second rotation axis 332 and spaced apart from the second rotation axis 332 by a certain distance (d) as shown in FIG. 9.

As a result, when the fourth driving motor 341 rotates by driving the controller 500, the second pinion gear 346 may transmit a driving force to the second rack gear 345 through rotation, and the second rack gear 345 may convert a rotational force of the second pinion gear 346 into a driving force for linear movement.

That is, the second rack gear 345 may convert a rotational force of the second pinion gear 346 into a direction parallel to the longitudinal direction of the second guide rail 343.

Through this, the second slider 344 may reciprocally move linearly along the second guide rail 343 through a driving force provided from the second rack gear 345. That is, the second slider 344 may reciprocally move linearly along the second guide rail 343 in a direction perpendicular to the second rotation axis 332.

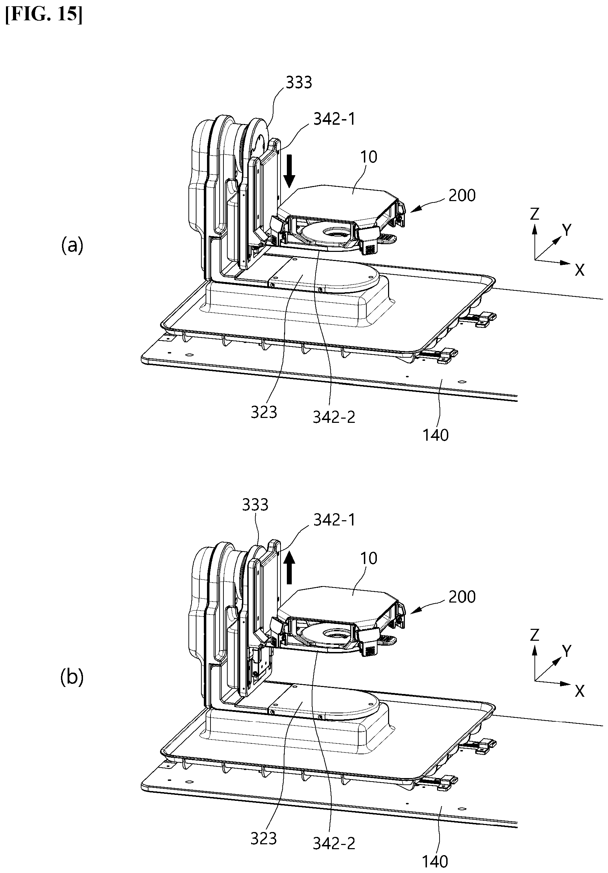

As a result, the holder support member 342 to which the second slider 344 is fixed on one side may reciprocally move linearly along the second guide rail 343 with respect to the second mounting member 333, as shown in FIG. 15. For reference, FIG. 15 is a drawing showing a state in which a holder support member moves linearly through a second linear driving part in the endoscopic surgery training apparatus according to an embodiment of the present invention.

In other words, the holder support member 342 may reciprocally move along a direction perpendicular to an axis that is parallel to the XY plane and perpendicular to the Z-axis. That is, the holder support member 342 may reciprocally move along a direction perpendicular to the second rotation axis 332 along the second guide rail 343 via the second slider 344.

Accordingly, the holder support member 342 may always move along a direction perpendicular to the second rotation axis 332 regardless of the rotation of the second mounting member 333.

That is, the second linear driving part 340 may implement a second linear movement of the holder support member 342.

Through this, the holder 200 coupled to the holder support member 342 and the training target member 10 mounted on the holder 200 may always move in a straight line along a direction perpendicular to the second rotation axis 332 regardless of the rotation of the second mounting member 333 together with the holder support member 342.

In other words, the first part 342-2 coupled to the holder 200 may always move in a straight line along a direction perpendicular to the second rotation axis 332 regardless of the rotation of the second mounting member 333 together with the second part 342-2.

Accordingly, the second linear driving part 340 may adjust a gap between the second rotation axis 332 and the first part 342-2 while maintaining the first part 342-2 and the second rotation axis 332 in a parallel state regardless of the rotation of the second mounting member 333 centered on the second rotation axis 332.

Through this, the second linear driving part 340 may adjust a rotation radius of the first part 342-2 centered on the second rotation axis 332 through the second linear movement of the first part 342-2.

In this way, in the endoscopic surgery training apparatus 1000 according to an embodiment of the present invention, the driving part 300 may be coupled such that the first linear driving part 310, the first rotational driving part 320, the second rotational driving part 330 and the second linear driving part 340 are interconnected, and the holder 200 may be mounted on the holder support member 342 constituting the second linear driving part 340.

Accordingly, the holder 200 may move in the same manner as the holder support member 342 through the driving of the driving part 300.

Through this, when the first linear driving part 310 is driven, if the moving member 312 moves linearly along the X-axis direction with respect to the support plate 140 together with the first slider 314, the holder 200 mounted on the holder supporting member 342 may move linearly along the X-axis direction with respect to the support plate 140 together with the holder supporting member 342. That is, the holder 200 mounted on the holder supporting member 342 may implement a first linear movement through the linear movement of the moving member 312 by the first linear driving part 310.

In addition, when the first rotational driving part 320 is driven, if the first mounting member 323 rotates about the first rotation axis 322 relative to the moving member 312, the holder 200 mounted on the holder support member 342 may rotate about the first rotation axis 322 together with the first mounting member 323. That is, the holder 200 mounted on the holder support member 342 may implement a first rotation through the rotation of the first mounting member 323 by the first rotational driving part 320.

Moreover, when the second rotational driving part 330 is driven, if the second mounting member 333 rotates about the second rotation axis 332 relative to the first mounting member 323, the holder 200 mounted on the holder support member 342 may rotate about the second rotation axis 332 together with the second mounting member 333. That is, the holder 200 mounted on the holder support member 342 may implement a second rotation through the rotation of the second mounting member 333 by the second rotational driving part 330.

In addition, when the second linear driving part 340 is driven, if the holder support member 342 moves linearly along a direction perpendicular to the second rotation axis 332 together with the second slider 344, the holder 200 coupled to the holder support member 342 may move linearly along a direction perpendicular to the second rotation axis 332 together with the holder support member 342. That is, the holder 200 mounted on the holder support member 342 may implement a second linear movement through the linear movement of the holder support member 342 by the second linear driving part 340.

Through this, the holder 200 mounted on the holder support member 342 in the endoscopic surgery training apparatus 1000 according to an embodiment of the present invention may implement a four-degree-of-freedom movement through the driving of the driving part 300.

That is, in the endoscopic surgery training apparatus 1000 according to an embodiment of the present invention, the holder 200 mounted on the holder support member 342 may perform a first linear movement through the first linear driving member 310, a first rotation through the first rotation driving member 320, a second rotation through the second rotation driving member 330, and a second linear movement through the second linear driving member 340.

In addition, when the holder support member 342 is placed at the initial position through the driving part 300 as described above, the center of the holder 200 may be placed so as to be in a straight line with the first rotation axis 322, which is the central axis for the first rotation of the holder support member 342.

Moreover, the first part 342-2 of the holder support member 342 to which the holder 200 is coupled may be maintained in a state of being spaced apart in parallel with the second rotation axis 332, which is the central axis for the second rotation of the holder support member 342, while the distance from the second rotation axis 332 may be adjusted.

Accordingly, the endoscopic surgery training apparatus 1000 according to an embodiment of the present invention may implement all four degrees of freedom of movement of the holder 200 mounted on the holder support member 342, thereby allowing the position of the holder 200 mounted on the holder support member 342 to be changed in various ways through a combination of the first linear movement of the holder support member 342 by the first linear driving part 310, the second linear movement of the holder support member 342 by the second linear driving part 340, the first rotation of the holder support member 342 by the first rotational driving part 320, and the second rotation of the holder support member 342 by the second rotational driving part 330.

Accordingly, when the position of the first part 342-2 is changed by the driving part 300 while the holder 200 is coupled to the first part 342-2 of the holder support member 342, the training target member 10 fixed to the holder 200 may implement all of various parts of the organ regardless of the shape of the organ, and the user may implement a specific part of the target organ similar to the actual environment by changing the position of the training target member 10 coupled to the holder 200.

That is, in the endoscopic surgery training apparatus 1000 according to an embodiment of the present invention, the training target member 10 fixed to the holder 200 may implement all of various parts of the organ regardless of the shape of the organ, and the user may implement a specific part of the target organ similar to the actual environment through the training target member 10.

For example, as shown in FIG. 8 and FIG. 11, the holder 200 may be mounted on the holder support member 342 of the driving part 300, and the holder support member 342 may be disposed to be positioned at the initial position through the driving part 300.

Herein, when the holder support member 342 is disposed to be positioned at the initial position, the first slider 314 may be moved to the leftmost position along the first guide rail 313, the rotation angle of the first mounting member 323 and the rotation angle of the second mounting member 333 may be 0 degrees, and the second slider 344 may be positioned as far away from the second rotation axis 332 as possible along the second guide rail 343.

In addition, the training target member 10 for implementing the stomach may be mounted on the holder 200.

Then, while the training target member 10 is mounted on the holder 200, the first mounting member 323 may be rotated 105 degrees clockwise around the first rotation axis 322 through the first rotation drive member 320, and the second mounting member 333 may be rotated 21 degrees clockwise around the second rotation axis 332 through the second rotation drive member 330.

Thereafter, the holder support member 342 may be moved linearly 36 mm in a direction closer to the second rotation axis 332 through the second linear drive member 340.

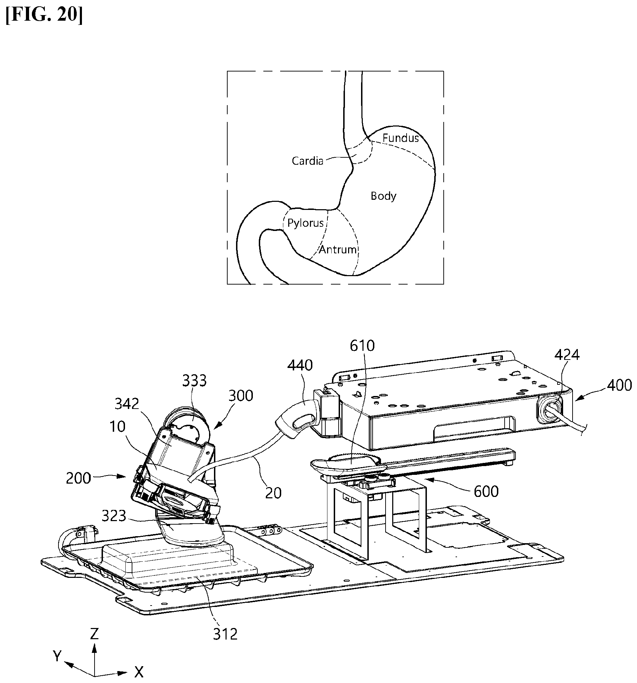

Through this, the holder 200 mounted on the holder support member 342 may be rotated 105 degrees clockwise around the first rotation axis 322 as shown in FIG. 20, rotated 21 degrees clockwise around the second rotation axis 332, and then moved 36 mm in a straight line in a direction closer to the second rotation axis 332.

For reference, FIG. 20 is a drawing showing a state in which the endoscopic surgery training apparatus according to an embodiment of the present invention is in use, showing a state in which a holder and a training target member are disposed to be positioned at different specific positions.

As a result, the holder 200 mounted on the holder support member 342 may be changed to a first simulation position corresponding to the greater curvature (GC) part of the antrum of the stomach in the training space (S1), and the training target member 10 mounted on the holder 200 changed to the first simulation position in the training space (S1) may implement the inner wall of the GC part of the antrum of the stomach.

That is, the endoscopic surgery training apparatus 1000 according to an embodiment of the present invention may implement the greater curvature (GC) part of the antrum of the stomach in the training space (S1) by changing the holder 200 mounted on the holder support member 342 to the first simulation position, and the training target member 10 mounted on the holder 200 may implement the inner wall of the GC part of the antrum of the stomach in the first simulation position.

As another example, as shown in FIG. 8 and FIG. 11, the holder 200 may be mounted on the holder support member 342 of the driving part 300, and the holder support member 342 may be disposed so as to be positioned at the initial position described above, and the training target member 10 for implementing camouflage may be mounted on the holder 200.

Then, the first mounting member 323 may be rotated 75 degrees counterclockwise around the first rotation axis 322 through the first rotation driving member 320, and the second mounting member 333 may be rotated 133 degrees clockwise around the second rotation axis 332 through the second rotation driving member 330.

Afterwards, the holder support member 342 may be linearly moved 28.5 mm in a direction closer to the second rotation axis 332 through the second linear driving member 340.

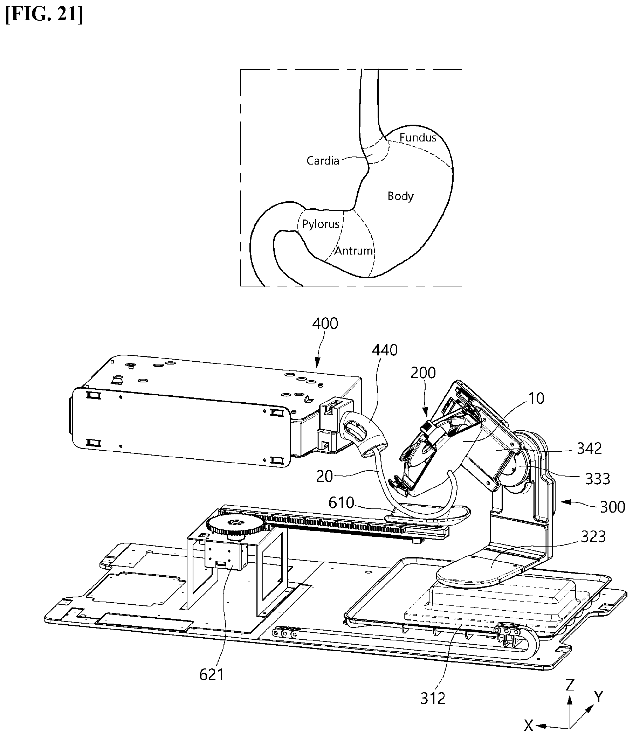

Through this, while the holder 200 mounted on the holder support member 342 is rotated 75 degrees clockwise around the first rotation axis 322 as shown in FIG. 21, it may be rotated 133 degrees counterclockwise around the second rotation axis 332 and then linearly moved 28.5 mm in a direction closer to the second rotation axis 332.

As a result, the holder 200 mounted on the holder support member 342 may be changed to a second simulation position corresponding to the lesser curvature (LC) part of the antrum of the stomach in the training space (S1), and the training target member 10 mounted on the holder 200 changed to the second simulation position in the training space (S1) may implement the inner wall of the LC part of the antrum of the stomach.

That is, the endoscopic surgery training apparatus 1000 according to an embodiment of the present invention may implement the lesser curvature (LC) part of the antrum of the stomach in the training space (S1) by changing the holder 200 mounted on the holder support member 342 to the second simulation position, and the training target member 10 mounted on the holder 200 may implement the inner wall of the LC part of the antrum of the stomach in the second simulation position.

As another example, as shown in FIG. 8 and FIG. 11, the holder 200 may be mounted on the holder support member 342 of the driving part 300, the holder support member 342 may be disposed to be positioned at the initial position described above, and the training target member 10 for implementing the colon may be mounted on the holder 200.

Then, the moving member 312 may be moved 61 mm along the X-axis direction through the first linear driving part 310, and the first mounting member 323 may be rotated 15 degrees clockwise around the first rotation axis 322 through the first rotational driving part 320.

Thereafter, the second mounting member 333 may be rotated 10 degrees clockwise around the second rotation axis 332 through the second rotation driving member 330, and the holder support member 342 may be linearly moved 15 mm in a direction closer to the second rotation axis 332 through the second linear driving member 340.

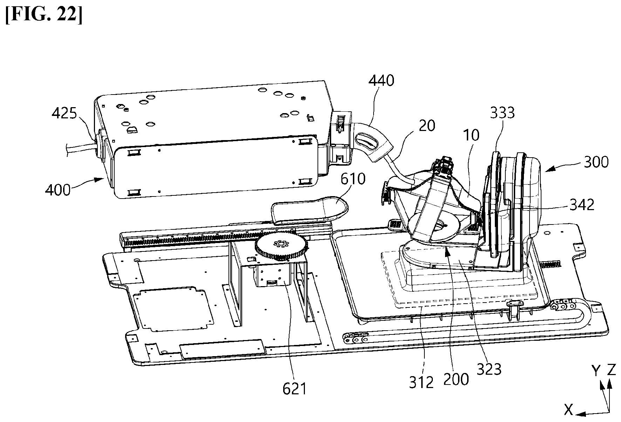

Through this, the holder 200 mounted on the holder support member 342 may be linearly moved 61 mm along the X-axis direction as shown in FIG. 22, rotated 15 degrees clockwise around the first rotation axis 322, rotated 10 degrees clockwise around the second rotation axis 332, and then linearly moved 15 mm in a direction closer to the second rotation axis 332.

As a result, the holder 200 mounted on the holder support member 342 may be changed to a third simulation position corresponding to the Downside part of the colon in the training space (S1), and the training target member 10 mounted on the holder 200 may implement the inner wall of the Downside part of the colon.

That is, the endoscopic surgery training apparatus 1000 according to an embodiment of the present invention may implement the Downside part of the colon in the training space (S1) by changing the holder 200 mounted on the holder support member 342 to the third simulation position, and the training target member 10 mounted on the holder 200 may implement the inner wall of the Downside part of the colon in the third simulation position.

In this way, the endoscopic surgery training apparatus 1000 according to an embodiment of the present invention may implement a specific part of a target organ in the training space (S1) similar to the real one by changing the simulation position of the holder 200 through the training target member 10 fixed to the holder 200 and the driving part 300.

Therefore, the user may conduct training to increase skill by using the training target member 10 implemented in the training space (S1) to simulate a specific part of a target organ.

In this case, the controller 500 may drive the driving part 300 such that the movement of the holder support member 342 can occur in a state where the training target member 10 mounted on the holder 200 is changed to a specific simulation position of the organ for training in the training space (S1) (e.g., one of the first simulation position, the second simulation position and the third simulation position).

In other words, the controller 500 may control the driving part 300 to perform a simulation operation that simulates the movement of an organ that can occur during an endoscopic surgery process while the training target member 10 is changed to a specific simulation position of an organ for training in the training space (S1).

The movements of an organ that can occur during an actual endoscopic surgery process are as follows.

For example, when air is injected into the interior of an organ during an actual endoscopic surgery process, the organ may expand and move such that the tip of the endoscopic device and the inner wall of the organ move away from each other.

For example, when air is sucked into the interior of an organ during an actual endoscopic surgery process, the organ may contract and move such that the tip of the endoscopic device and the inner wall of the organ move closer.

For example, an organ may move due to the patient's breathing during an actual endoscopic surgery process.

For example, an organ may move due to an event such as a patient's gagging or sneezing during an actual endoscopic surgery process.

The controller 500 may control at least one of the first linear driving part 310, the first rotational driving part 320, the second linear driving part 340 and the second rotational driving part 330 such that the training target member 10 performs a simulation movement that simulates the movement of an organ that may occur during the endoscopic surgery process described above.

For example, the controller 500 may drive the second linear driving part 340 such that the holder support member 342 moves linearly along a direction perpendicular to the second rotation axis 332 in a state where the training target member 10 mounted on the holder 200 is changed to a specific simulation position of an organ for training in the training space (S1) (e.g., one of the first simulation position, the second simulation position and the third simulation position).

Accordingly, the training target member 10 mounted on the holder 200 may perform a simulation movement of moving in a straight line along a direction perpendicular to the second rotation axis 332 together with the holder support member 342 by moving the holder support member 342.

Through this, when the tip of the endoscope device 20 is arranged on one side of the training target member 10 as shown in FIGS. 20 to 22, the training target member 10 may move in a direction closer to or further away from the tip of the endoscope device 20.

For reference, FIG. 21 is a drawing showing a state in which the endoscopic surgery training apparatus according to an embodiment of the present invention is in use, showing a state in which a holder and a training target member are disposed to be positioned at different specific positions, and FIG. 22 is a diagram showing a state in which the endoscopic surgery training apparatus according to an embodiment of the present invention is in use, showing a state in which a holder and a training target member are disposed to be positioned at other specific positions.

The movement of the training target member 10 moving in a straight line in a direction closer to the tip of the endoscope device 20 simulates the movement of injecting air into the interior of an organ during an actual endoscopic surgery to expand the organ, thereby bringing the tip of the endoscope device and the inner wall of the organ closer.

In addition, the movement of the training target member 10 moving in a straight line away from the tip of the endoscope device 20 simulates the movement of the tip of the endoscope device and the inner wall of the organ moving away by inhaling air inside the organ and contracting the organ during actual endoscopic surgery.

As a result, the endoscopic surgery training apparatus 1000 according to an embodiment of the present invention may identically implement an airinflation/deflation situation in which air is injected into the inside of an organ during actual endoscopic surgery to inflate or contract the organ by allowing the training target member 10 mounted on the holder 200 to move in a straight line through the second linear driving part 340 while the training target member 10 is changed to a specific simulated position of the organ for training in the training space (S1).

In this case, the controller 500 may control the driving part 300 such that at least one of the first linear movement by the first linear driving part 310, the first rotation by the first rotational driving part 320, and the second rotation by the second rotational driving part 330 can be performed together with the second linear movement by the second linear driving part 340 in a state where the training target member 10 mounted on the holder 200 is changed to a specific simulation position of an organ for training in the training space (S1).

Accordingly, the training target member 10 mounted on the holder 200 may be moved in a specific simulation position of an organ for training in the training space (S1) by the driving part 300 not only in a straight line but also in a normal direction or in a twisted direction, thereby implementing a more diverse air-inflation/deflation situation.

As another example, in the endoscopic surgery training apparatus 1000 according to an embodiment of the present invention, the controller 500 may control the driving part 300 such that the holder support member 342 may perform at least one movement among a first linear movement by the first linear driving part 310, a first rotation by the first rotational driving part 320, a second rotation by the second rotational driving part 330, and a second linear movement by the second linear driving part 340 while the training target member 10 mounted on the holder 200 is changed to a specific simulated position of an organ for training in the training space (S1).

Accordingly, the training target member 10 mounted on the holder 200 may perform a simulated movement together with the holder support member 342 through the movement of the holder support member 342.

In this case, with the tip of the endoscope device 20 placed on one side of the training target member 10, the training target member 10 may perform a movement that simulates the movement of an organ due to a patient's breathing or an event (ex. coughing, sneezing) during an actual endoscopic surgery process through the controller 500.

Therefore, the endoscopic surgery training apparatus 1000 according to an embodiment of the present invention may similarly implement the movement of an organ that occurs in various situations, such as breathing, nausea, sneezing and the like that may occur during an endoscopic surgery process, through the controller 500 in a state where the training target member 10 mounted on the holder 200 is changed to a specific simulated position of an organ for training in the training space (S1).

In the present invention, the movement of the training target member 10 mounted on the holder 200 through the driving part 300 in a state where the training target member 10 is changed to a specific simulation position of an organ for training in the training space (S1) may be performed by the controller 500 controlling the driving part 300 when the user operates the same, or may be performed by the controller 500 automatically controlling the driving part 300 based on input data.

The channel forming part 400 may form an entry path for an endoscopic device 20 entering the training space (S1) from the outside.

That is, as shown in FIGS. 20 to 22, the tip of the endoscope device 20 may enter the training space (S1) after passing through the channel forming part 400, and may move toward the training target member 10 fixed to the holder 200 in the training space (S1).

Accordingly, the endoscope device 20 that has entered the training space (S1) through the channel forming part 400 may be disposed such that the tip faces one surface of the training target member 10 that has been changed to a specific simulation position of an organ for training in the training space (S1).

Through this, the user may train endoscopic surgery in the training space (S1) by bringing the tip of the endoscope device 20 close to the training target member 10 that has been changed to a specific simulation position of an organ for training in the training space (S1).

In this case, the channel forming part 400 may form at least two entry paths.

For example, the channel forming part 400 may form a first entry path that simulates the entry path of the endoscope device 20 during an upper gastrointestinal endoscopy and a second entry path that simulates the entry path of the endoscope device 20 during a colonoscopy.

To this end, as shown in FIGS. 1 to 4, FIGS. 16 and 17, the channel forming part 400 may include a channel case 410 coupled to the housing 100, a path part 420 formed by being formed to be recessed in from one surface of the channel case 410 so as to form an entry path of the endoscope device 20, and a path maintenance member 430 formed in a hollow shape such that the tip of the endoscope device 20 can pass through.

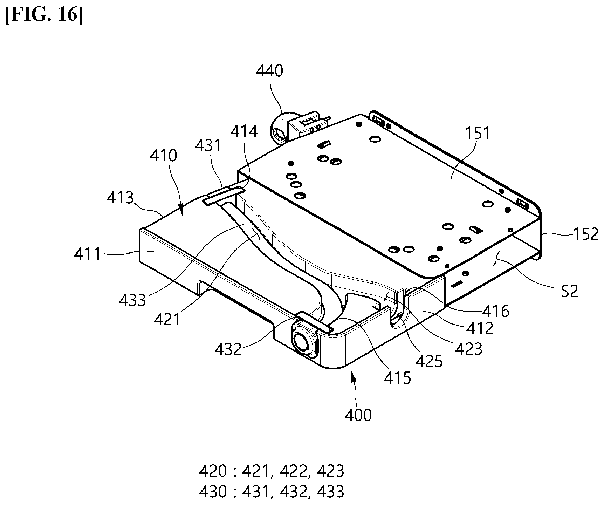

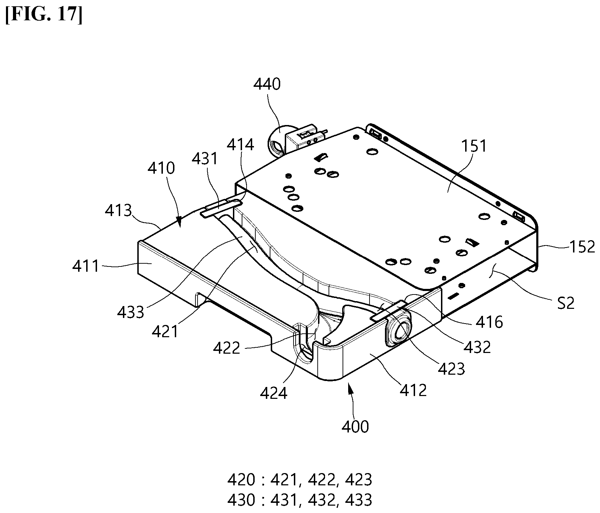

For reference, FIG. 16 is a drawing showing a channel forming part that is applicable to the endoscopic surgery training apparatus according to an embodiment of the present invention, showing a state in which a path maintenance member is mounted to be positioned at the first path forming part and the second path forming part, and FIG. 17 is a drawing showing a channel forming part that is applicable to the endoscopic surgery training apparatus according to an embodiment of the present invention, showing a state in which a path maintenance member is mounted to be positioned at the first path forming part and the third path forming part.

In this case, the path part 420 may include a first path forming part 421 formed such that one end is in communication with the training space (S1) and has a certain length, and a second path forming part 422 and a third path forming part 423 respectively extending from the first path forming part 421 so as to branch in different directions from the other end of the first path forming part 421.

In addition, the channel forming part 400 may include an exit forming member 440 coupled to the channel case 410 so as to be coupled to an end of the first path forming part 421, an end of the second path forming part 422 may be formed so as to be exposed to the outside to form a first entrance 424 through which the endoscope device 20 enters, and an end of the third path forming part 423 may also be formed so as to be exposed to the outside to form a second entrance 425 through which the endoscope device 20 enters.

Herein, the channel case 410 may include a first side surface 411 on which the first entrance 424 is formed, a second side surface 412 on which the second entrance 425 is formed, and a third side surface 413 on which the exit forming member 440 is coupled.

In addition, the first path forming part 421 and the second path forming part 422 may be arranged at a predetermined angle so as to simulate a path that is similar to a path connecting the throat and the esophagus through which the tip of the endoscope device 20 enters during an upper endoscopy, and the first path forming part 421 and the third path forming part 423 may be arranged to form an approximately straight line so as to simulate a path that is similar to a path connecting the anus and the rectum through which the tip of the endoscope device 20 enters during a colonoscopy.

Accordingly, when the path maintenance member 430 is disposed in the path part 420 so as to be positioned at the first path forming part 421 and the second path forming part 422 as shown in FIG. 16, the channel forming member 400 may form a first entry path that simulates the path through which the endoscope device 20 enters during an upper gastrointestinal endoscopy.

Through this, when the tip of the endoscope device 20 is inserted into the first entrance 424, the tip of the endoscope device 20 may move along the path maintenance member 430 forming the first entry path and then enter the training space (S1) through the exit forming member 440.

As a result, the operator may perform training in an environment similar to an actual gastroscopy by allowing the endoscope device 20 to enter the training space (S1) along a path similar to the path through which the endoscope device 20 enters during an actual gastroscopy through the channel forming part 400.

On the other hand, if the path maintenance member 430 is disposed in the path part 420 so as to be positioned at the first path forming part 421 and the third path forming part 423 as shown in FIG. 17, the channel forming part 400 may form a second entry path that simulates the path through which the endoscope device 20 enters during a colonoscopy.

Through this, when the tip of the endoscope device 20 is inserted into the second entrance 425, the tip of the endoscope device 20 may move along the path maintenance member 430 forming the second entry path and then enter the training space (S1) through the exit forming member 440.

As a result, the operator may perform training in an environment similar to an actual colonoscopy by allowing the endoscope device 20 to enter the training space (S1) along a path similar to the path through which the endoscope device 20 enters during an actual colonoscopy through the channel forming member 400.

In this case, the path maintenance member 430 may be detachably coupled to the path part 420, and the channel forming member 400 may be provided in a drawertype manner in which the channel case 410 is slidably coupled to the housing 100 along the rail 180.

Through this, the user may change the position of the path maintenance member 430 coupled to the path part 420 while the path part 420 is exposed to the outside by withdrawing the channel case 410 from the housing 100.

Therefore, the user may easily change the entry path of the endoscope device 20 entering the training space (S1) from the outside.

That is, the user may pull out the channel case 410 from the housing 100 and mount the path maintenance member 430 on the path part 420 such that the path part 420 is exposed to the outside, or mount the path maintenance member 430 on the path part 420 such that the path maintenance member 430 is positioned on the first path forming part 421 and the third path forming part 423.

Through this, the entry path of the endoscope device 20 formed through the path maintenance member 430 may be easily changed.

Then, when the user changes the position of the path maintenance member 430 coupled to the path part 420 and inserts the channel case 410 into the interior of the housing 100, the path maintenance member 430 may selectively connect the first entrance 424 to the exit forming member 440 or the second entrance 425 to the exit forming member 440.

To this end, the channel forming member 400 may be slidably coupled to a storage space (S2) of the housing 100 by the channel case 410, and the path maintenance member 430 may be detachably coupled to the path part 420 through the first coupling port 431 and the second coupling port 432 provided on each end side, respectively.

For example, the housing 100 may include a storage space (S2) formed to be adjacent to the training space (S1), and the storage space (S2) may be defined by a space forming member 151 having a cross-sectional shape roughly in the shape of the letter 'U' with three open sides as shown in FIGS. 2, 15, and 16, and a finishing plate 152 covering one side of the space forming member 151. In addition, the exit forming member 440 may be coupled to the space forming member 151 so as to be in communication with the storage space (S2).

Through this, the storage space (S2) may be formed in the housing 100 such that two side surfaces are open.

That is, the storage space (S2) may be formed such that a first corresponding side surface corresponding to the first side surface 411 and a second corresponding side surface corresponding to the second side surface 412 are open when the channel case 410 is inserted into the storage space (S2).

Accordingly, even if the channel case 410 is inserted into the storage space (S2), the first entrance 424 and the second entrance 425 may remain exposed to the outside through the first corresponding side surface and the second corresponding side surface of the storage space (S2), respectively.

As a result, the endoscope device 20 may enter the training space (S1) through the first entrance 424 or the second entrance 425, and the channel case 410 may be inserted into the storage space (S2) or withdrawn from the storage space (S2) through the first corresponding side surface.

In this case, the channel case 410 may be guided to slide through the rail 180. For example, the rail 180 may be formed to correspond to one surface of the channel case 410 and one surface of the space forming member 151 facing each other.

In addition, the path maintenance member 430 may include a hollow connecting pipe 433 having a predetermined length, a first coupling port 431 provided at one end of the connecting pipe 433 so as to be detachably coupled to one end of the first path forming part 421, and a second coupling port 432 provided at the other end of the connecting pipe 433 so as to be detachably coupled to one end of the second path forming part 422 or one end of the third path forming part 423, and the connecting pipe 433 may be made of a material whose shape can be easily changed by an external force.

In this case, the channel case 410 may include a first coupling groove 414 formed to have a shape corresponding to the first coupling hole 431 while being positioned at the end side of the first path forming part 421, a second coupling groove 415 formed to have a shape corresponding to the second coupling hole 432 while being positioned at the end side of the second path forming part 422, and a third coupling groove 41) formed to have a shape corresponding to the second coupling hole 432 while being positioned at the end side of the third path forming part 423.

Accordingly, the first coupling port 431 may be detachably coupled to the first coupling groove 414, and when the first coupling port 431 is coupled to the first coupling groove 414, the connecting pipe 433 may be communicated with the exit forming member 440.

In addition, the second coupling port 432 may be detachably coupled to the second coupling groove 415 or the third coupling groove 416, and when the second coupling port 432 is coupled to the second coupling groove 415, the connecting pipe 433 may be communicated at one end with the first entrance 424, and when the second coupling port 432 is coupled to the third coupling groove 416, the connecting pipe 433 may be communicated at one end with the second entrance 425.

Accordingly, while the first coupling port 431 is coupled to the first coupling groove 414, when the second coupling port 432 is selectively coupled to either the second coupling groove 415 or the third coupling groove 416, the path maintenance member 430 may form a first entry path or a second entry path.

Meanwhile, the endoscopic surgery training apparatus 1000 according to an embodiment of the present invention may further include an auxiliary support part 600 for supporting a part of the endoscopic device 20 that has entered the training space (S1) through the channel forming member 400.

Such an auxiliary support part 600 may include an auxiliary support 610 for supporting a part of the endoscope device 20, and may be disposed so as to be positioned at a bottom of the channel forming member 400, and when necessary, the auxiliary support 610 may move linearly toward the holder 200 along the X-axis direction.

That is, the auxiliary support 610 may move linearly toward the holder 200 while being positioned at a bottom of the channel forming member 400 only when it is necessary to support a part of the endoscope device 20 that has entered the training space (S1) from the channel forming member 400.

For example, during an actual endoscopic surgery, the endoscope device 20 that has entered the inside of an organ may maintain a state in which a part (ex., a curved part excluding the tip) is in contact with the inner wall of the organ depending on a specific part of the organ.

That is, when the tip of the endoscope device 20 approaches a specific part of an organ, a portion of the entire length may be maintained in a bent state, and the bent portion may be maintained in a state of contacting the inner wall of the organ.

In the present invention, by supporting a portion of the endoscope device 20 that has entered the training space (S1) through the auxiliary support 610, a state in which a portion of the endoscope device 20 that has entered the interior of an organ during an actual endoscopic surgery contacts the inner wall of the organ may be similarly implemented.

For example, as shown in FIG. 21, when the training target member 10 mounted on the holder 200 implements the inner wall of the lesser curvature (LC) portion of the Antrum of the stomach in the training space (S1), the auxiliary support 610 may move 190 mm linearly toward the holder 200 along the X-axis direction while being positioned at a bottom of the channel forming part 400 by driving the controller 500.

Accordingly, the auxiliary support 610 may support the bent portion of the endoscope device 20 that entered the training space (S1) through the channel forming part 400, thereby implementing a state in which the bent portion of the endoscope device 20 that entered the inside of the stomach during an actual gastrointestinal endoscopy comes into contact with the inner wall of the greater curvature (GC) portion of the antrum of the stomach.

On the other hand, as shown in FIG. 20, when the training target member 10 mounted on the holder 200 implements the inner wall of the GC portion of the antrum of the stomach in the training space (S1), the auxiliary support 610 may maintain the initial state of being positioned at a bottom of the channel forming part 400 without entering the training space (S1) side through the driving of the controller 500.

Through this, the auxiliary support 610 may move to the training space (S1) through the controller 500 only when necessary according to a specific portion of the organ implemented through the training target member 10 in the training space (S1).

To this end, the auxiliary support part 600 may include an auxiliary support 610 for supporting a part of the endoscope device 20 that has entered the training space (S1), and an auxiliary driving part 620 for changing the position of the auxiliary support 610 through a fifth driving motor 621 within the training space (S1).

In this case, the controller 500 may drive the auxiliary driving part 620 to move the auxiliary support 610 toward the training target member 10 when the training target member 10 is changed to a specific simulation position of an organ that has been input in the training space (S1).

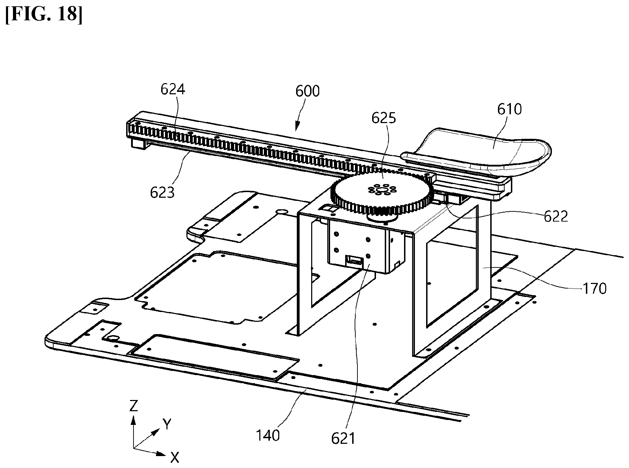

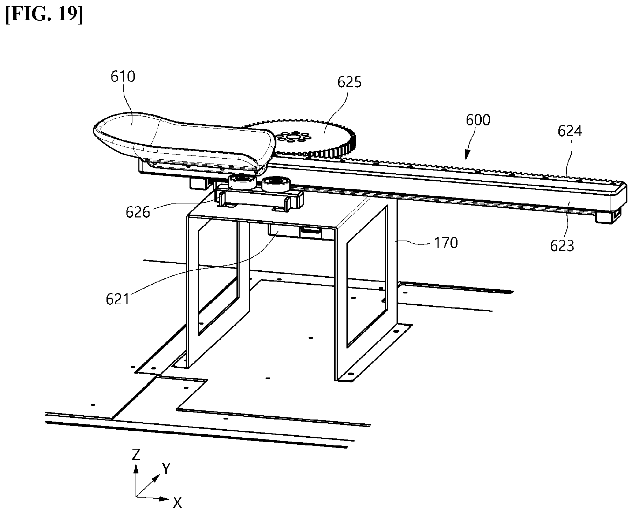

As a specific example, as shown in FIGS. 18 and 19, the auxiliary driving part 620 may include a third slider 622 fixed to the coupling member 170, and a third guide rail 623 movably coupled to the third slider 622 so as to be able to move linearly in the X-axis direction and having the auxiliary support 610 fixedly coupled to one side.

For reference, FIG. 18 is a drawing showing an auxiliary driving part that is applicable to the endoscopic surgery training apparatus according to an embodiment of the present invention, and FIG. 19 is a drawing looking at FIG. 18 from another direction.

The coupling member 170 may be coupled to the support plate 140, and the auxiliary driving part 620 may be spaced apart from the support plate 140 by a certain height such that the auxiliary driving part 620 may operate smoothly.

In addition, the auxiliary driving part 620 may further include a third rack gear 624 formed along the X-axis direction on one side of the third guide rail 623, and a third pinion gear 625 that is axially coupled to a rotation axis of the fifth driving motor 621 so as to be rotated by the fifth driving motor 621 and meshes with the third rack gear 624, and the fifth driving motor 621 may be fixedly coupled to the coupling member 170.

Moreover, the auxiliary driving part 620 may further include a guide roller 626 for guiding the sliding movement of the third guide rail 623, and the guide roller 626 may be coupled to the coupling member 170 so as to be positioned on the opposite side of the third rack gear 624 and contact one side of the third guide rail 623.

Accordingly, when the fifth driving motor 621 rotates through the driving of the controller 500, the third pinion gear 625 may transmit a driving force to the third rack gear 624 through rotation, and the third rack gear 624 may convert the rotational force of the third pinion gear 625 into a driving force for linear movement.

That is, the third rack gear 624 may convert the rotational power of the third pinion gear 625 in a direction parallel to the X-axis.

Through this, the third guide rail 623 may reciprocally move in a linear direction along the X-axis through the third slider 622 through the driving force provided by the third rack gear 624, and the third guide rail 623 may smoothly move in a linear direction through the guide roller 626.

As a result, the auxiliary support 610 fixed to one side of the third guide rail 623 may move in a linear direction along the X-axis to enter the training space (S1) and move toward the training target member 10 or maintain the initial state of being positioned at a bottom of the channel forming part 400.

Herein, the housing 100 may further include a placement space (S3) in which the auxiliary support part 600 is placed inside, and the placement space (S3) may be separated from the training space (S1) by a partition wall 160.

In addition, the placement space (S3) may be formed inside the housing 100 so as to be adjacent to the training space (S1) along the X-axis direction and positioned below the channel forming member 400.

Through this, when the training target member 10 fixed to the holder 200 is changed to a specific simulation position (e.g., the second simulation position) that requires the auxiliary support 610 among the specific simulation positions of an input organ, the controller 500 may drive the auxiliary driving part 620 to linearly move the auxiliary support 610 from the placement space (S3) to the training target member 10 arranged in the training space (S1).

On the other hand, when the training target member 10 fixed to the holder 200 is changed to a specific simulation position (e.g., the first simulation position or the third simulation position) that does not require the auxiliary support 610 among the specific simulation positions of an input organ, the controller 500 may maintain the auxiliary support 610 in the initial state of being placed in the placement space (S3).

Meanwhile, the controller 500 may control at least one of the first linear driving part 310, the first rotational driving part 320, the second linear driving part 340, the second rotational driving part 330 and the auxiliary driving part 620 such that the training target member 10 performs a simulation movement that simulates the movement of an organ that can occur in various situations such as breathing, gagging and sneezing during an endoscopic surgery process.

Meanwhile, the endoscopic surgery training apparatus 1000 according to an embodiment of the present invention may further include an operating part 700 for user operation.

Such an operating part 700 may generate a user input signal for changing the training target member 10 to a specific simulation position of an organ.

For example, the operating part 700 may be a known touch screen panel. In this case, the operating part 700 may be provided on one side of the housing 100 as shown in FIG. 1. Alternatively, the operating part 700 may be separated from the housing 100 and communicate with the controller 500 in a wired or wireless manner, although not shown.

As another example, the operating part 700 may be a known terminal that is capable of wired or wireless communication, such as a smart phone or tablet PC.

A user may transmit an input signal to the controller 500 through the operating part 700, and the controller 500 may control the overall operation including the driving part 300 and the auxiliary driving part 620 based on the input signal transmitted from the operating part 700.

As a non-limiting example, the user may select any one of the various simulation positions of various specific organs input through the operating part 700, and the controller 500 may drive the driving part 300 and the auxiliary driving part 620 to implement the simulation position of the specific organ selected by the user in the training space (S1) by using the training target member 10.

In addition, the user may also finely adjust the position of the holder 200 by directly manipulating the driving part 300 through the operating part 700.

Through this, the user may finely change the position of the training target member 10 by manipulating the operating part 700 while the training target member 10 is implemented as the simulation position of a specific organ in the training space (S1), thereby allowing the user to more accurately change the training target member 10 to an intended simulation position.

Meanwhile, referring to FIG. 4, the endoscopic surgery training apparatus 1000 according to an embodiment of the present invention may further include a connector 103 provided on one side of the housing 100. The connector 103 is electrically connected to the holder support member 342 by a wire (not shown). The wire (not shown) may be extended and arranged inside the housing 100 so as not to interfere with other components.

The holder 200 coupled to the holder support member 342 may be electrically connected to the connector 103. In this case, the contact portion of the holder support member 342 and the holder 200 may be electrically connected by having conductivity. In this case, the training target member 10 coupled to the holder 200 may be electrically connected to the connector 103.

The connector 103 is partially exposed to the outside of the housing 100 and is connected to a terminal (not shown) of a cable (not shown) that is electrically connected to a cauterizer (not shown). When the terminal (not shown) of the cable (not shown) is connected to the connector 103, the cauterizer may be electrically connected to the training target member 10 coupled to the holder 200 via the holder support member 342 and the holder 200.

**EXPLANATION OF REFERENCE NUMERALS**

| | | | |
|---|---|---|---|
| 10: | Training target member | 20: | Endoscopic device |
| 1000: | Endoscopic surgery training apparatus | | |
| 100: | Housing | 103: | Connector |
| 110: | First housing | 120: | Second housing |
| 130: | Door | 140: | Support plate |
| 151: | Space forming member | 152: | End plate |
| 160: | Partition wall | 170: | Coupling member |
| 180: | Rail | S1: | Training space |
| S2: | Storage space | S3: | Placement space |
| 200: | Holder | 210: | Holder body |
| 212: | Mounting part | 214: | Leg part |
| 215: | Protrusion | 220: | Grasping member |
| 230: | First magnet member | 240: | Second magnet member |
| 250: | Fourth magnet member | 300: | Driving part |
| 310: | First linear driving part | 311: | First driving motor |
| 312: | Moving member | 313: | First guide rail |
| 314: | First slider | 315: | First rack gear |
| 316: | First pinion gear | 320: | First rotational driving part |
| 321: | Second driving motor | 322: | First rotational axis |
| 323: | First mounting member | 323-1: | First-1 mounting member |
| 323-2: | First-2 mounting member | 324: | First driving gear |
| 325: | First driven gear | 330: | Second rotational driving part |
| 331: | Third driving motor | 332: | Second rotational axis |
| 333: | Second mounting member | 334: | Second driving gear |
| 335: | Second driven gear | 340: | Second linear drive part |
| 341: | Fourth driving motor | 342: | Holder support member |
| 342-1: | First part | 342-2: | Second part |
| 342a: | Settling groove | 343: | Second guide rail |
| 344: | Second slider | 345: | Second rack gear |
| 346: | Second pinion gear | 350: | Cover member |
| 360: | Third magnet member | 400: | Channel forming part |
| 410: | Channel case | 411: | First side surface |
| 412: | Second side surface | 413: | Third side surface |
| 414: | First coupling groove | 415: | Second coupling groove |
| 416: | Third coupling groove | 420: | Path part |
| 421: | First path forming part | 422: | Second path forming part |
| 423: | Third path forming part | 424: | First entrance |
| 425: | Second entrance | 430: | Path maintenance member |
| 431: | First coupling port | 432: | Second coupling port |
| 433: | Connecting pipe | 440: | Exit forming member |
| 500: | Controller | 600: | Auxiliary support part |
| 610: | Auxiliary support | 620: | Auxiliary driving part |
| 621: | Fifth driving motor | 622: | Third slider |
| 623: | Third guide rail | 624: | Third rack gear |
| 625: | Third pinion gear | 626: | Guide roller |
| 700: | Operating part | | |

## Claims

1. An endoscopic surgery training apparatus (1000), comprising:
a holder(200) configured to fix a training target member(10);
a driving part(300) comprising a holder support member(342) to which the holder(200) is coupled, and configured to change a position of the holder(200) coupled to the holder support member (342) within the training space (S1) through at least one degree-of-freedom movement so as to move the training target member (10) fixed to the holder (200) to a specific simulation position of an organ for training within the training space (S1); and
a controller (500) for controlling the driving part(300),
**characterized in that**
the apparatus further comprises:
a housing(100) comprising a training space(S1) formed inside for training a surgery, and a support plate(140) forming a bottom surface of the training space(S1); and
a channel forming part(400) formed in the housing(100) so as to form an entry path of an endoscopic device(20) entering the training space(S1) from the outside,
wherein the driving part(300) changes the position of the holder(200) coupled to the holder support member(342) within the training space(S1) through a four-degree-of-freedom movement including a first linear movement, a first rotation, a second rotation and a second linear movement,
wherein the driving part(300) comprises:
a first linear driving part(310) comprising a moving member(312) that reciprocates linearly along the X-axis with respect to the support plate(140) via a first driving motor (311);
a first rotational driving part(320) comprising a second driving motor(321) that is fixedly coupled to the moving member(312), a first rotation axis(322) that rotates around the Z-axis that is perpendicular to the X-axis with respect to the moving member(312) through a driving force of the second driving motor(321), and a first mounting member(323) that is fixedly coupled to the first rotation axis(322);
a second rotational driving part(330) comprising a third driving motor(331) that is fixedly coupled to the first mounting member(323), a second rotation axis(332) that rotates around an axis that is parallel to the XY plane and perpendicular to the Z axis with respect to the first mounting member(323) through a driving force of the third driving motor(331), and a second mounting member(333) that is fixedly coupled to the second rotation axis(332); and
a second linear driving part(340) comprising a fourth driving motor(341) that is fixedly coupled to the second mounting member(333), and provides a driving force for reciprocating a linear movement of the holder support member(342) along a direction perpendicular to an axis that is parallel to the XY plane and perpendicular to the Z-axis with respect to the second mounting member(333).

2. The endoscopic surgery training apparatus of claim 1,
wherein in the holder support member(342), a first part(342-2) to which the holder(200) is coupled is arranged so as to be parallel to the second rotation axis(332) and to be spaced apart from the second rotation axis(332) by a certain distance.

3. The endoscopic surgery training apparatus of claim 2, wherein when the holder support member(342) is placed in an initial position by the driving part(300) while the holder(200) is coupled to the first part(342-2), the center of the holder(200) is placed in a straight line with the first rotation axis(322), which is the central axis for a first rotation of the holder support member(342).

4. The endoscopic surgery training apparatus of any one of the preceding claims, wherein the holder(200) comprises a holder body(210) comprising a mounting part(212) that is detachably coupled to one side of the driving part(300) and a plurality of leg parts(214) that extend from the mounting part(212), and a plurality of gripping members(220) that are detachably coupled to the plurality of leg parts(214) so as to be able to grip the training target member(10),
wherein the holder(200) comprises a plurality of first magnet members(230) that are provided on each of the plurality of leg parts(214), and a plurality of second magnet members(240) that are provided on each of the plurality of gripping members(220) so as to correspond to the plurality of first magnet members(230), and
wherein each of the plurality of gripping members(220) is detachably coupled to the holder body(210) through a first magnet member(230) and a second magnet member(240) corresponding to each other.

5. The endoscopic surgery training apparatus of any one of the preceding claims, wherein the endoscopic surgery training apparatus further comprises a third magnet member(360) provided on the holder support member(342), and a fourth magnet member(250) provided on the holder(200) to correspond to the third magnet member(360), and
wherein the holder(200) is detachably coupled to the holder support member(342) via the third magnet member(360) and the fourth magnet member(250).

6. The endoscopic surgery training apparatus of any one of the preceding claims, wherein the first linear driving part(310) further comprises:
a first guide rail(313) fixed to the support plate(140);
a first slider(314) movably coupled to the first guide rail(313) so as to be able to move linearly in the X-axis direction along the first guide rail(313);
a first rack gear(315) fixedly coupled to one side of the moving member(312) so as to be parallel to the longitudinal direction of the first guide rail(313); and
a first pinion gear(316) meshed with the first rack gear(315) while being axially coupled to the first driving motor(311) so as to be able to rotate by the first driving motor(311), and
wherein the moving member(312) moves in a reciprocating linear manner along the X-axis with respect to the support plate(140) through the first slider(314) that is fixed to one side,
wherein the first rotational driving part(320) further comprises a first driving gear(324) that is axially coupled to the second driving motor(321), and a first driven gear(325) that is axially coupled to the first rotation axis(322) and meshes with the first drive gear, and
wherein the first mounting member(323) rotates around the Z-axis, which is perpendicular to the X-axis, with respect to the moving member(312) through a rotation of the first rotation axis(322),
wherein the second rotational driving part(330) further comprises a second driving gear (334) that is axially coupled to the third driving motor(331), and a second driven gear (335) that is axially coupled to the second rotation axis(332) and meshes with the second driving gear(334), and
wherein the second mounting member(333) rotates around an axis perpendicular to the Z-axis with respect to the first mounting member(323) through a rotation of the second rotation axis(332),
wherein the second linear driving part(340) further comprises:
a second guide rail(343) fixed to the second mounting member(333);
a second slider(344) movably coupled to the second guide rail(343) so as to be able to reciprocally move linearly along the second guide rail(343);
a second rack gear (345) fixedly coupled to one side of the holder support member(342) so as to be parallel to the longitudinal direction of the second guide rail(343); and
a second pinion gear(346) meshed with the second rack gear(345) while being axially coupled to the fourth driving motor(341) so as to be able to rotate by the fourth driving motor(341), and
wherein the holder support member(342) moves in a reciprocating linear manner along the second guide rail343) with respect to the second mounting member(333) through the second slider(344) that is fixed to one side.

7. The endoscopic surgery training apparatus of any one of the preceding claims, wherein the controller (500) drives the driving part(300) such that movement of the holder support member (342) occurs while the training target member(10) is moved to a specific simulation position of an organ for training.

8. The endoscopic surgery training apparatus of any one of the preceding claims, wherein the channel forming part(400) comprises:
a channel case(410) coupled to the housing(100);
a path part(420) formed to be recessed from one surface of the channel case(410) so as to form an entry path for the endoscopic device(20); and
a path maintenance member(430) formed in a hollow shape such that the endoscopic device(20) passes through and detachably coupled to the path part(420),
wherein the path part(420) comprises:
a first path forming part(421) formed to be connected to the training space(S1) and have a certain length; and
a second path forming part(422) and a third path forming part(423) each extending from the first path forming part(421) so as to branch in different directions from the other end of the first path forming part(421), and
wherein the path maintenance member(430) is mounted on the path part(420) so as to be positioned at the first path forming part(421) and the second path forming part(422) to form a first entry path, or is mounted on the path part(420) so as to be positioned at the first path forming part(421) and the third path forming part(423) to form a second entry path,
wherein the path maintenance member(430) comprises:
a hollow connecting pipe(433) having a predetermined length;
a first coupling port(431) provided at one end of the connecting pipe(433) so as to be detachably coupled to one end of the first path forming part(421); and
a second coupling port(432) provided at the other end of the connecting pipe(433) so as to be detachably coupled to one end of the second path forming part(422) or one end of the third path forming part(423).

9. The endoscopic surgery training apparatus of any one of the preceding claims, wherein the endoscopic surgery training apparatus further comprises:
an auxiliary support part(600) positioned at a bottom of the channel forming part(400) so as to support a portion of the endoscopic device(20) that has entered the training space(S1) through the channel forming part(400).

10. The endoscopic surgery training apparatus of claim 9, wherein the auxiliary support part(600) comprises an auxiliary support(610) configured to support a portion of an endoscopic device(20) that has entered the training space(S1), and an auxiliary driving part(620) configured to change a position of the auxiliary support(610) through a fifth driving motor(621) within the training space(S1), and
wherein the controller(500) drives the auxiliary driving part(620) to move the auxiliary support(610) toward the training target member(10) when the training target member(10) moves to a preset specific simulation position.

11. The endoscopic surgery training apparatus of claim 10, wherein the auxiliary driving part(620) further comprises:
a third slider(622) fixed to a coupling member(170) that is coupled to the support plate(140);
a third guide rail(623) that is movably coupled to the third slider(622) so as to be able to move linearly in the X-axis direction and has the auxiliary support(610) fixedly coupled to one side;
a third rack gear(624) formed along the X-axis direction on one side of the third guide rail(623); and
a third pinion gear(625) that is axially coupled to the fifth driving motor(621) so as to be able to rotate by the fifth driving motor(621) and meshes with the third rack gear(624), and
wherein the auxiliary support(610) moves toward the training target member(10) through a linear movement of the third guide rail(623).

12. The endoscopic surgery training apparatus of any one of the preceding claims, wherein the endoscopic surgery training apparatus further comprises:
an operating part(700) for user operation,
wherein the operating part(700) generates a user input signal to move the training target member(10) to a specific simulation position of an organ.

13. The endoscopic surgery training apparatus of any one of the preceding claims, wherein the endoscopic surgery training apparatus further comprises:
a connector(103) provided on one side of the housing(100) so as to be electrically connected to the holder support member(342), and a portion of which is exposed to the outside of the housing(100) and is coupled to a terminal of a cable that is electrically connected to a cautery, and
wherein the holder(200) that is coupled to the holder support member(342) is electrically connected to the connector(103).

## Patentansprüche

1. Eine endoskopische chirurgische Trainingsvorrichtung (1000), umfassend:
einen Halter (200), der konfiguriert ist, um ein Trainingszielelement (10) zu fixieren;
ein Antriebsteil (300), das ein Halterstützelement (342) umfasst, mit dem der Halter (200) gekoppelt ist, und konfiguriert ist, um eine Position des Halters (200), der mit dem Halterstützelement (342) gekoppelt ist, innerhalb des Trainingsraums (S1) durch mindestens eine Freiheitsgradbewegung zu ändern, um das an dem Halter (200) fixierte Trainingszielelement (10) zu einer spezifischen Simulations position eines Organs zum Trainieren innerhalb des Trainingsraums (S1) zu bewegen; und eine Steuerung (500) zum Steuern des Antriebsteils (300),
**dadurch gekennzeichnet, dass**
die Vorrichtung ferner umfasst:
ein Gehäuse (100), das einen Trainingsraum (S1), der im Inneren zum Trainieren einer Operation ausgebildet ist, und eine Stützplatte (140), die eine Bodenfläche des Trainingsraums (S1) bildet, umfasst; und
ein Kanalbilungsteil(400), das in dem Gehäuse (100) ausgebi Idet ist, um einen Eintrittsweg einer endoskopischen Vorrichtung (20) zu bilden, die von außen in den Trainingsraum (S1) eintritt,
wobei das Antriebsteil (300) die Position des Halters (200), der mit dem Halterstützelement (342) gekoppelt ist, innerhalb des Trainingsraums (S1) durch eine Vier-Freiheitsgradbewegung ändert, die eine erste lineare Bewegung, eine erste Drehung, eine zweite Drehung und eine zweite lineare Bewegung beinhaltet,
wobei das Antriebsteil (300) umfasst:
ein erstes lineares Antriebsteil (310), das ein Bewegungselement (312) umfasst, das sich linear entlang der X-Achse in Bezug auf die Stützplatte (140) über einen ersten Antriebsmotor (311) hin- und herbewegt;
ein erstes Drehantriebsteil (320), das einen zweiten Antriebsmotor (321), der fest mit dem Bewegungselement (312) gekoppelt ist, eine erste Drehachse (322), die sich um die Z-Achse dreht, die senkrecht zu der X-Achse in Bezug auf das Bewegungselement (312) ist, durch eine Antriebskraft des zweiten Antriebsmotors (321) und ein erstes Befestigungselement (323), das fest mit der ersten Drehachse (322) gekoppelt ist, umfasst;
ein zweites Drehantriebsteil (330), das einen dritten Antriebsmotor (331), der fest mit dem ersten Befestigungselement (323) gekoppelt ist, eine zweite Drehachse (332), die sich um eine Achse dreht, die parallel zu der XY-Ebene und senkrecht zu der Z-Achse in Bezug auf das erste Befestigungselement (323) ist, durch eine Antriebskraft des dritten Antriebsmotors (331) und ein zweites Befestigungselement (333), das fest mit der zweiten Drehachse (332) gekoppelt ist, umfasst; und
ein zweites lineares Antriebsteil (340), das einen vierten Antriebsmotor (341) umfasst, der fest mit dem zweiten Befestigungselement (333) gekoppelt ist und eine Antriebskraft zum Hin- und Herbewegen einer linearen Bewegung des Halterstützelements (342) entlang einer Richtung bereitstellt, die senkrecht zu einer Achse ist, die parallel zu der XY-Ebene und senkrecht zu der Z-Achse in Bezug auf das zweite Befestigungselement (333) ist.

2. Die endoskopische chirurgische Trainingsvorrichtung gemäß Anspruch 1,
wobei in dem Halterstützelement (342) ein erstes Teil (342-2), mit dem der Halter (200) gekoppelt ist, angeordnet ist, um parallel zu der zweiten Drehachse (332) zu sein und um einen bestimmten Abstand von der zweiten Drehachse (332) beabstandet zu sein.

3. Die endoskopische chirurgische Trainingsvorrichtung gemäß Anspruch 2, wobei, wenn das Halterstützelement (342) durch das Antriebsteil (300) in einer Anfangsposition platziert ist, während der Halter (200) mit dem ersten Teil (342-2) gekoppelt ist, die Mitte des Halters (200) in einer geraden Linie mit der ersten Drehachse (322) platziert ist, die die Mittelachse für eine erste Drehung des Halterstützelements (342) ist.

4. Die endoskopische chirurgische Trainingsvorrichtung gemäß einem der vorhergehenden Ansprüche, wobei der Halter (200) einen Halterkörper (210) umfasst, der ein Montageteil (212), das lösbar mit einer Seite des Antriebsteils (300) gekoppelt ist, und eine Vielzahl von Beinteilen (214), die sich von dem Montageteil (212) erstrecken, und eine Vielzahl von Greifelementen (220) umfasst, die lösbar mit der Vielzahl von Beinteilen (214) gekoppelt sind, um in der Lage zu sein, das Trainingszielelement (10) zu greifen,
wobei der Halter (200) eine Vielzahl von ersten Magnetelementen (230), die an jedem der Vielzahl von Beinteilen (214) bereitgestellt sind, und eine Vielzahl von zweiten Magnetelementen (240) umfasst, die an jedem der Vielzahl von Greifelementen (220) bereitgestellt sind, um der Vielzahl von ersten Magnetelementen (230) zu entsprechen, und
wobei jedes der Vielzahl von Greifelementen (220) durch ein erstes Magnetelement (230) und ein zweites Magnetelement (240), die einander entsprechen, lösbar mit dem Halterkörper (210) gekoppelt ist.

5. Die endoskopische chirurgische Trainingsvorrichtung gemäß einem der vorhergehenden Ansprüche, wobei die endoskopische chirurgische Trainingsvorrichtung ferner ein drittes Magnetelement (360), das an dem Halterstützelement (342) bereitgestellt ist, und ein viertes Magnetelement (250) umfasst, das an dem Halter (200) bereitgestellt ist, um dem dritten Magnetelement (360) zu entsprechen, und
wobei der Halter (200) über das dritte Magnetelement (360) und das vierte Magnetelement (250) lösbar mit dem Halterstützelement (342) gekoppelt ist.

6. Die endoskopische chirurgische Trainingsvorrichtung gemäß einem der vorhergehenden Ansprüche, wobei das erste lineare Antriebsteil (310) ferner umfasst:
eine erste Führungsschiene (313), die an der Stützplatte (140) befestigt ist;
einen ersten Schieber (314), der beweglich mit der ersten Führungsschiene (313) gekoppelt ist, um in der Lage zu sein, sich linear in der X-Achsenrichtung entlang der ersten Führungsschiene (313) zu bewegen;
eine erste Zahnstange (315), die fest mit einer Seite des Bewegungselements (312) gekoppelt ist, um parallel zu der Längsrichtung der ersten Führungsschiene (313) zu sein; und
ein erstes Ritzel (316), das mit der ersten Zahnstange (315) ineinandergreift, während es axial mit dem ersten Antriebsmotor (311) gekoppelt ist, um in der Lage zu sein, sich durch den ersten Antriebsmotor (311) zu drehen, und
wobei sich das Bewegungselement (312) in einer hin- und herbewegenden linearen Weise entlang der X-Achse in Bezug auf die Stützplatte (140) durch den ersten Schieber (314), der an einer Seite befestigt ist, bewegt,
wobei das erste Drehantriebsteil (320) ferner einen ersten Antrieb (324), der axial mit dem zweiten Antriebsmotor (321) gekoppelt ist, und einen ersten Abtrieb (325), der axial mit der ersten Drehachse (322) gekoppelt ist und mit dem ersten Antrieb ineinandergreift, umfasst, und
wobei sich das erste Befestigungselement (323) um die Z-Achse, die senkrecht zu der X-Achse ist, in Bezug auf das Bewegungselement (312) durch eine Drehung der ersten Drehachse (322) dreht,
wobei das zweite Drehantriebsteil (330) ferner einen zweiten Antrieb (334), der axial mit dem dritten Antriebsmotor (331) gekoppelt ist, und einen zweiten Abtrieb (335), der axial mit der zweiten Drehachse (332) gekoppelt ist und mit dem zweiten Antrieb (334) ineinandergreift, umfasst, und
wobei sich das zweite Befestigungselement (333) um eine Achse senkrecht zu der Z-Achse in Bezug auf das erste Befestigungselement (323) durch eine Drehung der zweiten Drehachse (332) dreht,
wobei das zweite lineare Antriebsteil (340) ferner umfasst:
eine zweite Führungsschiene (343), die an dem zweiten Befestigungselement (333) befestigt ist;
einen zweiten Schieber (344), der beweglich mit der zweiten Führungsschiene (343) gekoppelt ist, um in der Lage zu sein, sich linear entlang der zweiten Führungsschiene (343) hin- und herzubewegen;
eine zweite Zahnstange (345), die fest mit einer Seite des Halterstützelements (342) gekoppelt ist, um parallel zu der Längsrichtung der zweiten Führungsschiene (343) zu sein; und
ein zweites Ritzel (346), das mit der zweiten Zahnstange (345) ineinandergreift, während es axial mit dem vierten Antriebsmotor (341) gekoppelt ist, um in der Lage zu sein, sich durch den vierten Antriebsmotor (341) zu drehen, und
wobei sich das Halterstützelement (342) in einer hin- und herbewegenden linearen Weise entlang der zweiten Führungsschiene (343) in Bezug auf das zweite Befestigungselement (333) durch den zweiten Schieber (344), der an einer Seite befestigt ist, bewegt.

7. Die endoskopische chirurgische Trainingsvorrichtung gemäß einem der vorhergehenden Ansprüche, wobei die Steuerung (500) das Antriebsteil (300) derart antreibt, dass eine Bewegung des Halterstützelements (342) auftritt, während das Trainingszielelement (10) zu einer spezifischen Simulationsposition eines Organs zum Trainieren bewegt wird.

8. Die endoskopische chirurgische Trainingsvorrichtung gemäß einem der vorhergehenden Ansprüche, wobei das Kanalbildungsteil (400) umfasst:
ein Kanalgehäuse (410), das mit dem Gehäuse (100) gekoppelt ist;
ein Wegteil (420), das ausgebildet ist, um von einer Oberfläche des Kanalgehäuses (410) ausgespart zu sein, um einen Eintrittsweg für die endoskopische Vorrichtung (20) zu bilden; und
ein Wegaufrechterhaltungselement (430), das in einer hohlen Form ausgebildet ist, so dass die endoskopische Vorrichtung (20) durch das Wegteil (420) hindurchgeht und lösbar damit gekoppelt ist,
wobei das Wegteil (420) umfasst:
ein erstes Wegbildungsteil (421), das ausgebildet ist, um mit dem Trainingsraum (S1) verbunden zu sein und eine bestimmte Länge aufzuweisen; und
ein zweites Wegbildungsteil (422) und ein drittes Wegbildungsteil (423), die sich jewei Is von dem ersten Wegbilungsteil (421) erstrecken, um sich in unterschiedliche Richtungen von dem anderen Ende des ersten Wegbildungsteils (421) zu verzweigen, und
wobei das Wegaufrechterhaltungselement (430) an dem Wegteil (420) befestigt ist, um an dem ersten Wegbildungsteil (421) und dem zweiten Wegbildungsteil (422) positioniert zu sein, um einen ersten Eintrittsweg zu bilden, oder an dem Wegteil (420) befestigt ist, um an dem ersten Wegbildungsteil (421) und dem dritten Wegbildungsteil (423) positioniert zu sein, um einen zweiten Eintrittsweg zu bilden,
wobei das Wegaufrechterhaltungselement (430) umfasst:
ein hohles Verbindungsrohr (433), das eine vorbestimmte Länge aufweist;
einen ersten Kopplungsanschluss (431), der an einem Ende des Verbindungsrohrs (433) vorgesehen ist, um lösbar mit einem Ende des ersten Wegbildungsteil (421) gekoppelt zu sein; und
einen zweiten Kopplungsanschluss (432), der an dem anderen Ende des Verbindungsrohrs (433) vorgesehen ist, um lösbar mit einem Ende des zweiten Wegbi Idungstei Is (422) oder einem Ende des dritten Wegbildungsteils (423) gekoppelt zu sein.

9. Die endoskopische chirurgische Trainingsvorrichtung gemäß einem der vorhergehenden Ansprüche, wobei die endoskopische chirurgische Trainingsvorrichtung ferner umfasst:
ein Hilfsstützteil (600), das an einem Boden des Kanalbildungsteils (400) positioniert ist, um einen Abschnitt der endoskopischen Vorrichtung (20) zu stützen, der durch das Kanalbildungsteil (400) in den Trainingsraum (S1) eingetreten ist.

10. Die endoskopische chirurgische Trainingsvorrichtung gemäß Anspruch 9, wobei das Hilfsstützteil (600) eine Hilfsstütze (610), die konfiguriert ist, um einen Abschnitt einer endoskopischen Vorrichtung (20) zu stützen, der in den Trainingsraum (S1) eingetreten ist, und ein Hilfsantriebsteil (620) umfasst, das konfiguriert ist, um eine Position der Hilfsstütze (610) durch einen fünften Antriebsmotor (621) innerhalb des Trainingsraums (S1) zu ändern, und
wobei die Steuerung (500) das Hilfsantriebsteil (620) antreibt, um die Hilfsstütze (610) in Richtung des Trainingszielelements (10) zu bewegen, wenn sich das Trainingszielelement (10) zu einer voreingestellten spezifischen Simulationsposition bewegt.

11. Die endoskopische chirurgische Trainingsvorrichtung gemäß Anspruch 10, wobei das Hilfsantriebsteil (620) ferner umfasst:
einen dritten Schieber (622), der an einem Kopplungselement (170) befestigt ist, das mit der Stützplatte (140) gekoppelt ist;
eine dritte Führungsschiene (623), die beweglich mit dem dritten Schieber (622) gekoppelt ist, um in der Lage zu sein, sich linear in der X-Achsenrichtung zu bewegen, und die Hilfsstütze (610) fest mit einer Seite gekoppelt hat:
eine dritte Zahnstange (624), die entlang der X-Achsenrichtung auf einer Seite der dritten Führungsschiene (623) ausgebildet ist; und
ein drittes Ritzel (625), das axial mit dem fünften Antriebsmotor (621) gekoppelt ist, um in der Lage zu sein, sich durch den fünften Antriebsmotor (621) zu drehen, und mit der dritten Zahnstange (624) ineinandergreift, und
wobei sich die Hilfsstütze (610) in Richtung des Trainingszielelements (10) durch eine lineare Bewegung der dritten Führungsschiene (623) bewegt.

12. Die endoskopische chirurgische Trainingsvorrichtung gemäß einem der vorhergehenden Ansprüche, wobei die endoskopische chirurgische Trainingsvorrichtung ferner umfasst:
ein Bedienteil (700) zur Benutzerbedienung,
wobei das Bedienteil (700) ein Benutzereingabesignal erzeugt, um das Trainingszielelement (10) zu einer spezifischen Simulations position eines Organs zu bewegen.

13. Die endoskopische chirurgische Trainingsvorrichtung gemäß einem der vorhergehenden Ansprüche, wobei die endoskopische chirurgische Trainingsvorrichtung ferner umfasst:
einen Verbinder (103), der an einer Seite des Gehäuses (100) vorgesehen ist, um elektrisch mit dem Halterstützelement (342) verbunden zu sein, und von dem ein Abschnitt zu der Außenseite des Gehäuses (100) freiliegt und mit einem Anschluss eines Kabels gekoppelt ist, das elektrisch mit einer Kauterei verbunden ist, und
wobei der Halter (200), der mit dem Halterstützelement (342) gekoppelt ist, elektrisch mit dem Verbinder (103) verbunden ist.

## Revendications

1. Appareil d'entraînement à la chirurgie endoscopique (1000), comprenant :
un support (200) configuré pour fixer un élément cible d'entraînement (10) ; une partie d'entraînement (300) comprenant un élément de support de support (342) auquel le support (200) est couplé, et configurée pour changer une position du support (200) couplé à l'élément de support de support (342) à l'intérieur de l'espace d'entraînement (S1) par un mouvement selon au moins un degré de liberté de manière à déplacer l'élément cible d'entraînement (10) fixé au support (200) jusqu'à une position de simulation spécifique d'un organe pour l'entraînement à l'intérieur de l'espace d'entraînement (S1) ; et
un dispositif de commande (500) pour commander la partie d'entraînement (300),
**caractérisé en ce que**
l'appareil comprend en outre :
un boîtier (100) comprenant un espace d'entraînement (S1) formé à l'intérieur pour l'entraînement à une chirurgie, et une plaque de support (140) formant une surface inférieure de l'espace d'entraînement (S1) ; et
une partie de formation de canal (400) formée dans le boîtier (100) de manière à former un trajet d'entrée d'un dispositif endoscopique (20) entrant dans l'espace d'entraînement (S1) depuis l'extérieur,
dans lequel la partie d'entraînement (300) change la position du support (200) couplé à l'élément de support de support (342) à l'intérieur de l'espace d'entraînement (S1) par un mouvement de quatre degrés de liberté comprenant un premier mouvement linéaire, une première rotation, une seconde rotation et un second mouvement linéaire,
dans lequel la partie d'entraînement (300) comprend :
une première partie d'entraînement linéaire (310) comprenant un élément mobile (312) qui effectue un mouvement de va-et-vient linéaire le long de l'axe X par rapport à la plaque de support (140) via un premier moteur d'entraînement (311) ;
une première partie d'entraînement en rotation (320) comprenant un deuxième moteur d'entraînement (321) qui est couplé de manière fixe à l'élément mobile (312), un premier axe de rotation (322) qui tourne autour de l'axe Z qui est perpendiculaire à l'axe X par rapport à l'élément mobile (312) par une force d'entraînement du deuxième moteur d'entraînement (321), et un premier élément de montage (323) qui est couplé de manière fixe au premier axe de rotation (322) ;
une seconde partie d'entraînement en rotation (330) comprenant un troisième moteur d'entraînement (331) qui est couplé de manière fixe au premier élément de montage (323), un second axe de rotation (332) qui tourne autour d'un axe qui est parallèle au plan XY et perpendiculaire à l'axe Z par rapport au premier élément de montage (323) par une force d'entraînement du troisième moteur d'entraînement (331), et un second élément de montage (333) qui est couplé de manière fixe au second axe de rotation (332) ; et
une seconde partie d'entraînement linéaire (340) comprenant un quatrième moteur d'entraînement (341) qui est couplé de manière fixe au second élément de montage (333), et fournit une force d'entraînement pour effectuer un mouvement de va-et-vient linéaire de l'élément de support de support (342) le long d'une direction perpendiculaire à un axe qui est parallèle au plan XY et perpendiculaire à l'axe Z par rapport au second élément de montage (333).

2. Appareil d'entraînement à la chirurgie endoscopique selon la revendication 1,
dans lequel, dans l'élément de support de support (342), une première partie (342-2) à laquelle le support (200) est couplé est agencée de manière à être parallèle au second axe de rotation (332) et à être espacée du second axe de rotation (332) d'une certaine distance.

3. Appareil d'entraînement à la chirurgie endoscopique selon la revendication 2, dans lequel, lorsque l'élément de support de support (342) est placé dans une position initiale par la partie d'entraînement (300) alors que le support (200) est couplé à la première partie (342-2), le centre du support (200) est placé en ligne droite avec le premier axe de rotation (322), qui est l'axe central pour une première rotation de l'élément de support de support (342).

4. Appareil d'entraînement à la chirurgie endoscopique selon l'une quelconque des revendications précédentes, dans lequel le support (200) comprend un corps de support (210) comprenant une partie de montage (212) qui est couplée de manière détachable à un côté de la partie d'entraînement (300) et une pluralité de parties de jambe (214) qui s'étendent à partir de la partie de montage (212), et une pluralité d'éléments de préhension (220) qui sont couplés de manière détachable à la pluralité de parties de jambe (214) de manière à pouvoir saisir l'élément cible d'entraînement (10),
dans lequel le support (200) comprend une pluralité de premiers éléments d'aimant (230) qui sont prévus sur chacune des parties de jambe (214), et une pluralité de seconds éléments d'aimant (240) qui sont prévus sur chacun des éléments de préhension (220) de manière à correspondre à la pluralité de premiers éléments d'aimant (230), et
dans lequel chacun des éléments de préhension (220) est couplé de manière détachable au corps de support (210) par l'intermédiaire d'un premier élément d'aimant (230) et d'un second élément d'aimant (240) correspondant l'un à l'autre.

5. Appareil d'entraînement à la chirurgie endoscopique selon l'une quelconque des revendications précédentes, dans lequel l'appareil d'entraînement à la chirurgie endoscopique comprend en outre un troisième élément d'aimant (360) prévu sur l'élément de support de support (342), et un quatrième élément d'aimant (250) prévu sur le support (200) pour correspondre au troisième élément d'aimant (360), et
dans lequel le support (200) est couplé de manière détachable à l'élément de support de support (342) par l'intermédiaire du troisième élément d'aimant (360) et du quatrième élément d'aimant (250).

6. Appareil d'entraînement à la chirurgie endoscopique selon l'une quelconque des revendications précédentes, dans lequel la première partie d'entraînement linéaire (310) comprend en outre :
un premier rail de guidage (313) fixé à la plaque de support (140) ;
un premier coulisseau (314) couplé de manière mobile au premier rail de guidage (313) de manière à pouvoir se déplacer linéairement dans la direction de l'axe X le long du premier rail de guidage (313) ;
un premier engrenage à crémaillère (315) couplé de manière fixe à un côté de l'élément mobile (312) de manière à être parallèle à la direction longitudinale du premier rail de guidage (313) ; et
un premier engrenage à pignon (316) engrené avec le premier engrenage à crémaillère (315) tout en étant couplé axialement au premier moteur d'entraînement (311) de manière à pouvoir tourner par le premier moteur d'entraînement (311), et
dans lequel l'élément mobile (312) se déplace d'une manière linéaire en va-et-vient le long de l'axe X par rapport à la plaque de support (140) par l'intermédiaire du premier coulisseau (314) qui est fixé à un côté,
dans lequel la première partie d'entraînement en rotation (320) comprend en outre un premier engrenage d'entraînement (324) qui est couplé axialement au deuxième moteur d'entraînement (321), et un premier engrenage entraîné (325) qui est couplé axialement au premier axe de rotation (322) et s'engrène avec le premier engrenage d'entraînement, et
dans lequel le premier élément de montage (323) tourne autour de l'axe Z, qui est perpendiculaire à l'axe X, par rapport à l'élément mobile (312) par une rotation du premier axe de rotation (322),
dans lequel la seconde partie d'entraînement en rotation (330) comprend en outre un second engrenage d'entraînement (334) qui est couplé axialement au troisième moteur d'entraînement (331), et un second engrenage entraîné (335) qui est couplé axialement au second axe de rotation (332) et s'engrène avec le second engrenage d'entraînement (334), et
dans lequel le second élément de montage (333) tourne autour d'un axe perpendiculaire à l'axe Z par rapport au premier élément de montage (323) par une rotation du second axe de rotation (332),
dans lequel la seconde partie d'entraînement linéaire (340) comprend en outre :
un second rail de guidage (343) fixé au second élément de montage (333) ;
un second coulisseau (344) couplé de manière mobile au second rail de guidage (343) de manière à pouvoir se déplacer en va-et-vient linéairement le long du second rail de guidage (343) ;
un second engrenage à crémaillère (345) couplé de manière fixe à un côté de l'élément de support de support (342) de manière à être parallèle à la direction longitudinale du second rail de guidage (343) ; et
un second engrenage à pignon (346) engrené avec le second engrenage à crémaillère (345) tout en étant couplé axialement au quatrième moteur d'entraînement (341) de manière à pouvoir tourner par le quatrième moteur d'entraînement (341), et
dans lequel l'élément de support de support (342) se déplace d'une manière linéaire en va-et-vient le long du second rail de guidage (343) par rapport au second élément de montage (333) par l'intermédiaire du second coulisseau (344) qui est fixé à un côté.

7. Appareil d'entraînement à la chirurgie endoscopique selon l'une quelconque des revendications précédentes, dans lequel le dispositif de commande (500) entraîne la partie d'entraînement (300) de sorte qu'un mouvement de l'élément de support de support (342) se produise alors que l'élément cible d'entraînement (10) est déplacé jusqu'à une position de simulation spécifique d'un organe pour l'entraînement.

8. Appareil d'entraînement à la chirurgie endoscopique selon l'une quelconque des revendications précédentes, dans lequel la partie de formation de canal (400) comprend :
un boîtier de canal (410) couplé au boîtier (100) ;
une partie de trajet (420) formée pour être en retrait d'une surface du boîtier de canal (410) de manière à former un trajet d'entrée pour le dispositif endoscopique (20) ; et
un élément de maintien de trajet (430) formé dans une forme creuse de sorte que le dispositif endoscopique (20) passe à travers celui-ci, et couplé de manière détachable à la partie de trajet (420),
dans lequel la partie de trajet (420) comprend :
une première partie de formation de trajet (421) formée pour être reliée à l'espace d'entraînement (S1) et avoir une certaine longueur ; et
une deuxième partie de formation de trajet (422) et une troisième partie de formation de trajet (423) s'étendant chacune à partir de la première partie de formation de trajet (421) de manière à se ramifier dans différentes directions à partir de l'autre extrémité de la première partie de formation de trajet (421), et
dans lequel l'élément de maintien de trajet (430) est monté sur la partie de trajet (420) de manière à être positionné au niveau de la première partie de formation de trajet (421) et de la deuxième partie de formation de trajet (422) pour former un premier trajet d'entrée, ou est monté sur la partie de trajet (420) de manière à être positionné au niveau de la première partie de formation de trajet (421) et de la troisième partie de formation de trajet (423) pour former un second trajet d'entrée,
dans lequel l'élément de maintien de trajet (430) comprend :
un tuyau de raccordement creux (433) ayant une longueur prédéterminée ;
un premier orifice de couplage (431) prévu au niveau d'une extrémité du tuyau de raccordement (433) de manière à être couplé de manière détachable à une extrémité de la première partie de formation de trajet (421) ; et
un second orifice de couplage (432) prévu au niveau de l'autre extrémité du tuyau de raccordement (433) de manière à être couplé de manière détachable à une extrémité de la deuxième partie de formation de trajet (422) ou à une extrémité de la troisième partie de formation de trajet (423).

9. Appareil d'entraînement à la chirurgie endoscopique selon l'une quelconque des revendications précédentes, dans lequel l'appareil d'entraînement à la chirurgie endoscopique comprend en outre :
une partie de support auxiliaire (600) positionnée au niveau d'un fond de la partie de formation de canal (400) de manière à supporter une partie du dispositif endoscopique (20) qui est entrée dans l'espace d'entraînement (S1) à travers la partie de formation de canal (400).

10. Appareil d'entraînement à la chirurgie endoscopique selon la revendication 9, dans lequel la partie de support auxiliaire (600) comprend un support auxiliaire (610) configuré pour supporter une partie d'un dispositif endoscopique (20) qui est entrée dans l'espace d'entraînement (S1), et une partie d'entraînement auxiliaire (620) configurée pour changer une position du support auxiliaire (610) par l'intermédiaire d'un cinquième moteur d'entraînement (621) à l'intérieur de l'espace d'entraînement (S1), et
dans lequel le dispositif de commande (500) entraîne la partie d'entraînement auxiliaire (620) pour déplacer le support auxiliaire (610) vers l'élément cible d'entraînement (10) lorsque l'élément cible d'entraînement (10) se déplace jusqu'à une position de simulation spécifique prédéfinie.

11. Appareil d'entraînement à la chirurgie endoscopique selon la revendication 10, dans lequel la partie d'entraînement auxiliaire (620) comprend en outre :
un troisième coulisseau (622) fixé à un élément de couplage (170) qui est couplé à la plaque de support (140) ;
un troisième rail de guidage (623) qui est couplé de manière mobile au troisième coulisseau (622) de manière à pouvoir se déplacer linéairement dans la direction de l'axe X et a le support auxiliaire (610) couplé de manière fixe à un côté ;
un troisième engrenage à crémaillère (624) formé le long de la direction de l'axe X sur un côté du troisième rail de guidage (623) ; et
un troisième engrenage à pignon (625) qui est couplé axialement au cinquième moteur d'entraînement (621) de manière à pouvoir tourner par le cinquième moteur d'entraînement (621) et s'engrène avec le troisième engrenage à crémaillère (624), et
dans lequel le support auxiliaire (610) se déplace vers l'élément cible d'entraînement (10) par un mouvement linéaire du troisième rail de guidage (623).

12. Appareil d'entraînement à la chirurgie endoscopique selon l'une quelconque des revendications précédentes, dans lequel l'appareil d'entraînement à la chirurgie endoscopique comprend en outre :
une partie d'actionnement (700) pour l'actionnement de l'utilisateur,
dans lequel la partie d'actionnement (700) génère un signal d'entrée d'utilisateur pour déplacer l'élément cible d'entraînement (10) jusqu'à une position de simulation spécifique d'un organe.

13. Appareil d'entraînement à la chirurgie endoscopique selon l'une quelconque des revendications précédentes, dans lequel l'appareil d'entraînement à la chirurgie endoscopique comprend en outre :
un connecteur (103) prévu sur un côté du boîtier (100) de manière à être électriquement connecté à l'élément de support de support (342), et dont une partie est exposée à l'extérieur du boîtier (100) et est couplée à une borne d'un câble qui est électriquement connecté à un cautère, et
dans lequel le support (200) qui est couplé à l'élément de support de support (342) est électriquement connecté au connecteur (103).
